# EUROPEAN PATENT APPLICATION

(11) **EP 3 751 003 A1**
(43) Date of publication of application: **16.12.2020**
(21) Application number: 19179803.2
(22) Date of filing: 12.06.2019
(51) Int. Cl.: C12P 19/18, C12N 9/10, C12N 9/12, A61K 35/741, A61K 31/702, C07H 3/06

(54) **PRODUCTION OF FUCOSYLATED OLIGOSACCHARIDES IN BACILLUS**

(71) Applicant: Jennewein Biotechnologie GmbH, 53619 Rheinbreitbach (DE)
(72) Inventor: JENNEWEIN, Stefan, 53605 Bad Honnef (DE); WARTENBERG, Dirk, 55435 Gau-Algesheim (DE); HABERSETZER, Stefanie, 53604 Bad Honnef (DE)

(57) **Abstract**

Disclosed are non-sporulating *Bacillus* cells for the production of a fucosylated oligosaccharide like fucosylated lactose as well as a method for producing a fucosylated oligosaccharide, wherein said *Bacillus* cell has been genetically engineered to possess a lactose permease, a GDP-fucose biosynthesis pathway and a fucosyltransferase.

SEQUENCE LISTING

<110>  Jennewein Biotechnologie GmbH

<120>  Producing fucosylated oligosaccharides in Bacillus

<130>  P 1903 EP

<160>  5

<170>  PatentIn version 3.5

<210>  1
<211>  11270
<212>  DNA
<213>  Artificial Sequence

<220>
<223>  Suicide plasmid

<400>  1
ttctcatgtt tgacagctta tcatcgataa gctttaatgc ggtagtttat cacagttaaa      60

ttgctaacgc agtcaggcac cgtgtatgaa atctaacaat gcgctcatcg tcatcctcgg     120

caccgtcacc ctggatgctg taggcatagg cttggttatg ccggtactgc cgggcctctt     180

gcgggatatc gtccattccg acagcatcgc cagtcactat ggcgtgctgc tagcgctata     240

tgcgttgatg caatttctat gcgcacccgt tctcggagca ctgtccgacc gctttggccg     300

ccgcccagtc ctgctcgctt cgctacttgg agccactatc gactacgcga tcatggcgac     360

cacacccgtc ctgtggatcc tctacgccgg acgcatcgtg gccggcatca ccggcgccac     420

aggtgcggtt gctggcgcct atatcgccga catcaccgat ggggaagatc gggctcgcca     480

cttcgggctc atgagcgctt gtttcggcgt gggtatggtg gcaggccccg tggccggggg     540

actgttgggc gccatctcct tgcatgcacc attccttgcg gcggcggtgc tcaacggcct     600

caacctacta ctgggctgct tcctaatgca ggagtcgcat aagggagagc gtcgaccgat     660

gcccttgaga gccttcaacc cagtcagctc cttccggtgg gcgcggggca tgactatcgt     720

cgccgcactt atgactgtct tctttatcat gcaactcgta ggacaggtgc cggcagcgct     780

ctgggtcatt ttcggcgagg accgctttcg ctggagcgcg acgatgatcg gcctgtcgct     840

tgcggtattc ggaatcttgc acgccctcgc tcaagccttc gtcactggtc ccgccaccaa     900

acgtttcggc gagaagcagg ccattatcgc cggcatggcg gccgacgcgc tgggctacgt     960

cttgctggcg ttcgcgacgc gaggctggat ggccttcccc attatgattc ttctcgcttc    1020

cggcggcatc gggatgcccg cgttgcaggc catgctgtcc aggcaggtag atgacgacca    1080

tcagggacag cttcaaggat cgctcgcggc tcttaccagc ctaacttcga tcactggacc    1140

gctgatcgtc acggcgattt atgccgcctc ggcgagcaca tggaacgggt tggcatggat    1200

tgtaggcgcc gccctatacc ttgtctgcct ccccgcgttg cgtcgcggtg catggagccg    1260

ggccacctcg acctgaatgg aagccggcgg cacctcgcta acggattcac cactccaaga 1320

attggagcca atcaattctt gcggagaact gtgaatgcgc aaaccaaccc ttggcagaac 1380

atatccatcg cgtccgccat ctccagcagc cgcacgcggc gcatctcggg cagcgttggg 1440

tcctggccac gggtgcgcat gatcgtgctc ctgtcgttga ggacccggct aggctggcgg 1500

ggttgcctta ctggttagca gaatgaatca ccgatacgcg agcgaacgtg aagcgactgc 1560

tgctgcaaaa cgtctgcgac ctgagcaaca acatgaatgg tcttcggttt ccgtgtttcg 1620

taaagtctgg aaacgcggaa gtcagcgccc tgcaccatta tgttccggat ctgcatcgca 1680

ggatgctgct ggctaccctg tggaacacct acatctgtat taacgaagcg ctggcattga 1740

ccctgagtga tttttctctg gtcccgccgc atccataccg ccagttgttt accctcacaa 1800

cgttccagta accgggcatg ttcatcatca gtaacccgta tcgtgagcat cctctctcgt 1860

ttcatcggta tcattacccc catgaacaga aatccccctt acacggaggc atcagtgacc 1920

aaacaggaaa aaaccgccct taacatggcc cgctttatca gaagccagac attaacgctt 1980

ctggagaaac tcaacgagct ggacgcggat gaacaggcag acatctgtga atcgcttcac 2040

gaccacgctg atgagcttta ccgcagctgc ctcgcgcgtt tcggtgatga cggtgaaaac 2100

ctctgacaca tgcagctccc ggagacggtc acagcttgtc tgtaagcgga tgccgggagc 2160

agacaagccc gtcagggcgc gtcagcgggt gttggcgggt gtcggggcgc agccatgacc 2220

cagtcacgta gcgatagcgg agtgtatact ggcttaacta tgcggcatca gagcagattg 2280

tactgagagt gcaccagcgg gcaaggaaag ccttcaatat gtgcagtggt atgtcaactc 2340

tatgaagatc agcctgttta caatggcagg gtctttgctc tgtgtgacgt ttacggccta 2400

tgcgttttcg cgctttcggt ttaaagggag gaaatacgct ttaacgctct ttttattgct 2460

gcagatgatt cctcagtttt cagctttaat tgccttgttt gtgctggcgc aaatcttggg 2520

aatgatcaat agccactggc tgctaatctt gctttatatc ggcggcctga tcccgatgaa 2580

tacgtatttg atgaaagggt acatggattc cattccgatg gatttagacg aaagcgccaa 2640

gattgacgga gccagcagca ccagaatctt cttccagatc attctgccat tatcaaaacc 2700

gatggcggca gtcgtggcca tgaacggctt taccggtccg ctcggagatt ttgtgctgtc 2760

ctcaaccata ttgagaacgc ctgaatcata tacattgccc gtcggtctat tcaatttagt 2820

gaatgatgtc atgggggcca gctatacgac atttgcggcc ggagccctgc ttatcagcat 2880

accggttgcc gtcatcttta ttatgctgca aaagaatttt gtgtccggat taaccgcagg 2940

cggaacgaag ggctaagaga acaaggagga gaatgtgatg tcaaagcttg aaaaaacgca 3000

cgtaacaaaa gcaaaattta tgctccatgg gggagactac aaccccgatc agtggctgga 3060

tcggcccgat attttagctg acgatatcaa actgatgaag ctttctcata cgaatacgtt 3120

ttctgtcggc accagtgaat tcgagctcgg tacctaccgt tcgtataatg tatgctatac 3180

gaagttatta cgcgttaacc cgggcccgcg gatgcatatg atcagatcct ttaactctgg 3240

caaccctcaa aattgaatga gacatgctac acctccggat aataaatata tataaacgta 3300

tatagatttc ataaagtcta acacactaga cttatttact tcgtaattaa gtcgttaaac 3360

cgtgtgctct acgaccaaaa ctataaaacc tttaagaact ttcttttttt acaagaaaaa 3420

agaaattaga taaatctctc atatctttta ttcaataatc gcatccgatt gcagtataaa 3480

tttaacgatc actcatcatg ttcatattta tcagagctcg tgctataatt atactaattt 3540

tataaggagg aaaaaatatg ggcattttta gtatttttgt aatcagcaca gttcattatc 3600

aaccaaacaa aaaataagtg gttataatga atcgttaata agcaaaattc atataaccaa 3660

attaaagagg gttataatga acgagaaaaa tataaaacac agtcaaaact ttattacttc 3720

aaaacataat atagataaaa taatgacaaa tataagatta aatgaacatg ataatatctt 3780

tgaaatcggc tcaggaaaag gccattttac ccttgaatta gtaaagaggt gtaatttcgt 3840

aactgccatt gaaatagacc ataaattatg caaaactaca gaaaataaac ttgttgatca 3900

cgataatttc caagttttaa acaaggatat attgcagttt aaatttccta aaaaccaatc 3960

ctataaaata tatggtaata taccttataa cataagtacg gatataatac gcaaaattgt 4020

ttttgatagt atagctaatg agatttattt aatcgtggaa tacgggtttg ctaaaagatt 4080

attaaataca aaacgctcat tggcattact tttaatggca gaagttgata tttctatatt 4140

aagtatggtt ccaagagaat attttcatcc taaacctaaa gtgaatagct cacttatcag 4200

attaagtaga aaaaaatcaa gaatatcaca caaagataaa caaaagtata attatttcgt 4260

tatgaaatgg gttaacaaag aatacaagaa aatatttaca aaaaatcaat ttaacaattc 4320

cttaaaacat gcaggaattg acgatttaaa caatattagc tttgaacaat tcttatctct 4380

tttcaatagc tataaattat ttaataagta agttaaggga tgcataaact gcatccctta 4440

acttgttttt cgtgtgccta ttttttgtga atcgattata acttcgtata atgtatgcta 4500

tacgaacggt aggatcctct agagtcgacc tgcaggcatt ttacattttt agaaatgggc 4560

gtgaaaaaaa gcgcgcgatt atgtaaaata taaagattaa ctaataagga ggacaaacat 4620

ggcacaatct aaactgtatc cggttgtgat ggcgggcgga tctggatcaa gactgtggcc 4680

gctttcacgc gttctttacc cgaaacaatt tctgtgtctg aaaggagatt taacaatgct 4740

gcagacaaca atttgcagat taaatggcgt ggaatgtgaa tctccggtcg ttatttgcaa 4800

cgaacagcat agatttatcg tcgctgaaca acttcgccag ctgaacaaac tgacagaaaa 4860

catcatcctg gaaccggccg gcagaaatac agcaccggcg attgctcttg cagcgttagc 4920

tgccaaacgc cattcaccgg aaagcgatcc gcttatgtta gtgctggcag cggatcatgt 4980

cattgcggat gaagatgctt ttagagctgc cgttcgcaac gctatgccgt atgccgaagc 5040

aggcaaactt gtgacatttg gaattgtccc ggatttaccg gaaacaggct atggatatat 5100

cagacgcggc gaagtgtcag cgggagaaca agatatggtt gcctttgaag tggcacagtt 5160

tgtcgaaaaa ccgaatctgg aaacagcgca agcgtatgtt gcttcaggcg aatattattg 5220

gaacagcgga atgtttcttt ttagagccgg ccgctatctg gaagaactta aaaaatacag 5280

accggatatt ttagatgcat gtgaaaaagc gatgtcagct gtcgatccgg atttaaactt 5340

tatcagagtt gatgaagaag cctttctggc atgcccggaa gaaagcgttg attatgcagt 5400

gatggaacgc acagccgatg cagtggtcgt tccgatggat gcgggctggt ctgatgttgg 5460

atcatggtca agcctttggg aaattagcgc gcatacagct gaaggcaatg tttgtcatgg 5520

agatgtgatc aaccataaaa cagaaaacag ctatgtttac gcggaatctg gcctggtgac 5580

aacagtcgga gttaaagatt tagtggtcgt tcagacaaaa gatgcggtgt taattgctga 5640

tagaaacgcc gtgcaagatg ttaagaaagt tgtcgaacag atcaaagcag atggaagaca 5700

tgaacataga gtccatcgcg aagtttatcg cccgtgggga aaatatgata gcattgatgc 5760

gggcgatcgc tatcaagtca aacgcatcac agttaaaccg ggcgaaggac tgtctgttca 5820

gatgcatcat catcgcgcag aacattgggt tgtggtcgcc ggcacagcaa aagtgacaat 5880

tgatggcgat attaaactgc ttggagaaaa cgaatctatt tatatcccgt taggagctac 5940

acattgcctg gaaaacccgg gcaaaattcc gcttgattta atcgaagtca gaagcggatc 6000

ttatcttgaa gaagatgatg ttgtgcgctt tgccgataga tatggccgcg tttaagctct 6060

taaggaggat tttagaatga aaaaacttac atgctttaaa gcgtatgata ttcgcggcaa 6120

acttggagaa gaattaaacg aagatattgc ttggagaatc ggccgcgcct atggagaatt 6180

tctgaaaccg aaaacaatcg tgctgggcgg agatgtcaga cttacatcag aaacacttaa 6240

actggctctg gccaaaggcc tgcaagatgc tggagttgat gtgcttgata ttggcatgag 6300

cggaacagaa gaaatctatt ttgccacatt tcatttagga gtcgatggcg gaatcgaagt 6360

tacagcaagc cataatccga tggattataa cggaatgaaa ctggttagag aaggcgcaag 6420

accgatttct ggcgatacag gattaagaga tgtgcagcgc ctggcagaag cgaacgattt 6480

tccgccggtc gatgaaacaa aaagaggacg ctatcaacag attaatttac gcgatgcgta 6540

tgttgatcat ctttttggct acatcaacgt taaaaacctg acaccgctta aactggtgat 6600

caattctggc aacggagcag cgggaccggt tgtggatgct attgaagcca gatttaaagc 6660

acttggcgca ccggttgaat taatcaaagt gcataataca ccggatggca attttccgaa 6720

cggcattccg aacccgctgc ttccggaatg cagagatgat acacgcaatg cggttattaa 6780

acatggcgct gatatgggaa tcgcctttga tggagatttt gatcgctgtt ttctttttga 6840

tgaaaaaggc caatttatcg aaggatacta catcgtgggc ttactggctg aagcatttct 6900

ggagaaaaat ccgggagcga aaattatcca tgatccgaga ctttcttgga atacagtcga 6960

tgtcgttaca gctgccggcg gaacaccggt tatgtcaaaa acaggccatg cttttatcaa 7020

agaaagaatg cgcaaagaag atgcaatcta tggcggagaa atgagcgcgc atcattactt 7080

tagagatttt gcttactgcg attctggcat gatcccgtgg cttttagtcg cggaactggt 7140

ttgtcttaaa gataaaacac tgggagaact ggtcagagat cgcatggcag cgtttccggc 7200

ttcaggcgaa attaatagca aactggcaca accggtggaa gcgatcaaca gagtcgaaca 7260

gcatttttca cgcgaagcac tggcggttga tagaacagat ggaattagca tgacatttgc 7320

cgattggaga tttaatcttc gcacatctaa cacagaaccg gtggtcagac ttaacgttga 7380

atcacgcggc gatgtgccgt taatggaagc aagaacacgc acactgctta cattactgaa 7440

tgaataaaaa ggaggaacta ctatgacaac agaaccgctg tttttcaaac cggtctttaa 7500

agaaagaatt tggggcggaa cagctctggc cgattttggc tatacaatcc cgtctcaacg 7560

cacaggagaa tgctgggctt ttgcagcgca tcaaaatggc cagagcgttg tgcagaacgg 7620

catgtacaaa ggatttacac tgtctgaact gtgggaacat catagacatc tttttggcca 7680

attagaagga gatcgctttc cgctgcttac aaaaatttta gatgctgatc aggatctgtc 7740

agttcaggtt catccgaatg atgaatatgc caatatccat gaaaacggcg aacttggaaa 7800

aacagaatgc tggtatatta tcgattgtca gaaagatgct gaaattatct atggccataa 7860

cgccacaaca aaagaagaat taacaacaat gatcgaaaga ggagaatggg atgaattact 7920

gagacgcgtg aaagtcaaac cgggcgattt cttttatgtc ccgtcaggaa cagttcatgc 7980

aattggcaaa ggaatccttg cgttagaaac acaacagaat agcgatacaa catacagact 8040

gtacgattac gatcgcaaag atgcagaagg caaacttaga gaactgcatc ttaaaaaatc 8100

aatcgaagtt atcgaagtgc cgagcattcc ggaacgccat acagttcatc atgaacaaat 8160

cgaagattta ctgacaacaa cactgatcga atgcgcatat tttagcgtgg gcaaatggaa 8220

cctttcagga agcgcgtctt taaaacaaca gaaaccgttt ctgcttattt ctgtgatcga 8280

aggcgaagga cgcatgattt caggcgaata tgtctatccg tttaagaaag gcgatcacat 8340

gttactgccg tatggcctgg gagaatttaa acttgaagga tatgcggaat gtatcgtttc 8400

tcatctttaa gcgaggcagg atcaggacaa tgattatatt tttgtcatga atttcacgga 8460

agaaaaacag ctggtcacgt ttgatcagag tgtgaaggac ataatgacag gagacatatt 8520

gtcaggcgac ctgacgatgg aaaagtatga agtgagaatt gtcgtaaaca cacattaggc 8580

tgatgctccg ctcgatatgg gcggattctt ttttctatag aatgaaaacg cttgctaagt 8640

cttgggggga tgaaatcatg aaaagcaaag tgaaaatgtt ctttgcggct gccatcgtgt 8700

ggagtgcatg tagttcaaca ggatatgccg ctgccattga gaaggagaag cacgtgtcag 8760

agcttcgggc agaggatctt tttgttaaaa aagtagaggg gatgaacaag gattttatca 8820

aaggggcaga tgtatccagc gttattgctt tggaaaacag cggtgtcacc ttttacaata 8880

caaacggaaa acgccaggat atctttacaa ctttaaaaca ggctggggtc aactatgttc 8940

gcgtccgcat ctggaatcac ccgtatgatt caaatggcaa cgggtatggc gggggaaaca 9000

atgatgttca aaaagccatc gaaatcggaa aaagagcgac agcgaacgga atgaaggtgc 9060

tggccgactt tcactactct gatttctggg ccgatccagc gaaacaaaag gtgcccaaag 9120

cctgggcgaa tctcagcttt gaagcaaaaa aagcaaagct ctatgagtat acgaaacaaa 9180

gcctgcaaaa gatgatcaag gaaggcgtgc ggtgtgaaat accgcacaga tgcgtaagga 9240

gaaaataccg catcaggcgc tcttccgctt cctcgctcac tgactcgctg cgctcggtcg 9300

ttcggctgcg gcgagcggta tcagctcact caaaggcggt aatacggtta tccacagaat 9360

caggggataa cgcaggaaag aacatgtgag caaaaggcca gcaaaaggcc aggaaccgta 9420

aaaaggccgc gttgctggcg tttttccata ggctccgccc ccctgacgag catcacaaaa 9480

atcgacgctc aagtcagagg tggcgaaacc cgacaggact ataaagatac caggcgtttc 9540

cccctggaag ctccctcgtg cgctctcctg ttccgaccct gccgcttacc ggatacctgt 9600

ccgcctttct cccttcggga agcgtggcgc tttctcatag ctcacgctgt aggtatctca 9660

gttcggtgta ggtcgttcgc tccaagctgg gctgtgtgca cgaacccccc gttcagcccg 9720

accgctgcgc cttatccggt aactatcgtc ttgagtccaa cccggtaaga cacgacttat 9780

cgccactggc agcagccact ggtaacagga ttagcagagc gaggtatgta ggcggtgcta 9840

cagagttctt gaagtggtgg cctaactacg gctacactag aaggacagta tttggtatct 9900

gcgctctgct gaagccagtt accttcggaa aaagagttgg tagctcttga tccggcaaac 9960

aaaccaccgc tggtagcggt ggtttttttg tttgcaagca gcagattacg cgcagaaaaa 10020

aaggatctca agaagatcct ttgatctttt ctacggggtc tgacgctcag tggaacgaaa 10080

actcacgtta agggattttg gtcatgagat tatcaaaaag gatcttcacc tagatccttt 10140

taaattaaaa atgaagtttt aaatcaatct aaagtatata tgagtaaact tggtctgaca 10200

gttaccaatg cttaatcagt gaggcaccta tctcagcgat ctgtctattt cgttcatcca 10260

tagttgcctg actccccgtc gtgtagataa ctacgatacg ggagggctta ccatctggcc 10320

ccagtgctgc aatgataccg cgagacccac gctcaccggc tccagattta tcagcaataa 10380

accagccagc cggaagggcc gagcgcagaa gtggtcctgc aactttatcc gcctccatcc 10440

agtctattaa ttgttgccgg gaagctagag taagtagttc gccagttaat agtttgcgca 10500

acgttgttgc cattgctgca ggcatcgtgg tgtcacgctc gtcgtttggt atggcttcat 10560

tcagctccgg ttcccaacga tcaaggcgag ttacatgatc ccccatgttg tgcaaaaaag 10620

cggttagctc cttcggtcct ccgatcgttg tcagaagtaa gttggccgca gtgttatcac 10680

tcatggttat ggcagcactg cataattctc ttactgtcat gccatccgta agatgctttt 10740

ctgtgactgg tgagtactca accaagtcat tctgagaata gtgtatgcgg cgaccgagtt 10800

gctcttgccc ggcgtcaaca cgggataata ccgcgccaca tagcagaact ttaaaagtgc 10860

tcatcattgg aaaacgttct tcggggcgaa aactctcaag gatcttaccg ctgttgagat 10920

ccagttcgat gtaacccact cgtgcaccca actgatcttc agcatctttt actttcacca 10980

gcgtttctgg gtgagcaaaa acaggaaggc aaaatgccgc aaaaaaggga ataagggcga 11040

cacggaaatg ttgaatactc atactcttcc tttttcaata ttattgaagc atttatcagg 11100

gttattgtct catgagcgga tacatatttg aatgtattta gaaaaataaa caaatagggg 11160

ttccgcgcac atttccccga aaagtgccac ctgacgtcta agaaaccatt attatcatga 11220

cattaaccta taaaaatagg cgtatcacga ggccctttcg tcttcaagaa 11270

<210> 2
<211> 12427
<212> DNA
<213> Artificial Sequence

<220>
<223> expression plasmid

<400> 2
   ttaagttatt ggtatgactg gttttaagcg caaaaaaagt tgctttttcg tacctattaa 60
   tgtatcgttt tagaaaaccg actgtaaaaa gtacagtcgg cattatctca tattataaaa 120
   gccagtcatt aggcctatct gacaattcct gaatagagtt cataaacaat cctgcatgat 180
   aaccatcaca aacagaatga tgtacctgta aagatagcgg taaatatatt gaattacctt 240
   tattaatgaa ttttcctgct gtaataatgg gtagaaggta attactatta ttattgatat 300
   ttaagttaaa cccagtaaat gaagtccatg gaataataga aagagaaaaa gcattttcag 360
   gtataggtgt tttgggaaac aatttccccg aaccattata tttctctaca tcagaaaggt 420
   ataaatcata aaactctttg aagtcattct ttacaggagt ccaaatacca gagaatgttt 480
   tagatacacc atcaaaaatt gtataaagtg gctctaactt atcccaataa cctaactctc 540
   cgtcgctatt gtaaccagtt ctaaaagctg tatttgagtt tatcaccctt gtcactaaga 600
   aaataaatgc agggtaaaat ttatatcctt cttgttttat gtttcggtat aaaacactaa 660
   tatcaatttc tgtggttata ctaaaagtcg tttgttggtt caaataatga ttaaatatct 720
   cttttctctt ccaattgtct aaatcaattt tattaaagtt catttgatat gcctcctaaa 780
   tttttatcta aagtgaattt aggaggctta cttgtctgct ttcttcatta gaatcaatcc 840
   ttttttaaaa gtcaatatta ctgtaacata aatatatatt ttaaaaatat cccactttat 900
   ccaattttcg tttgttgaac taatgggtgc tttagttgaa gaataaagac cacattaaaa 960
   aatgtggtct tttgtgtttt tttaaaggat ttgagcgtag cgaaaaatcc ttttctttct 1020
   tatcttgata ataagggtaa ctattgccga tcgtccattc cgacagcatc gccagtcact 1080
   atggcgtgct gctagcgcca ttcgccattc aggctgcgca actgttggga agggcgatcg 1140
   gtgcgggcct cttcgctatt acgccagctg gcgaaagggg gatgtgctgc aaggcgatta 1200
   agttgggtaa cgccagggtt ttcccagtca cgacgttgta aaacgacggc cagtgaattc 1260
   gagctcaggc cttaactcac attaattgcg ttgcgctcac tgcccgcttt ccagtcggga 1320
   aacctgtcgt gccagctgca ttaatgaatc ggccaacgcg cggggagagg cggtttgcgt 1380
   attgggcgcc agggtggttt ttcttttcac cagtgagacg ggcaacagct gattgccctt 1440
   caccgcctgg ccctgagaga gttgcagcaa gcggtccacg ctggtttgcc ccagcaggcg 1500
   aaaatcctgt ttgatggtgg ttgacggcgg gatataacat gagctgtctt cggtatcgtc 1560
   gtatcccact accgagatat ccgcaccaac gcgcagcccg gactcggtaa tggcgcgcat 1620
   tgcgcccagc gccatctgat cgttggcaac cagcatcgca gtgggaacga tgccctcatt 1680
   cagcatttgc atggtttgtt gaaaaccgga catggcactc cagtcgcctt cccgttccgc 1740
   tatcggctga atttgattgc gagtgagata tttatgccag ccagccagac gcagacgcgc 1800
   cgagacagaa cttaatgggc ccgctaacag cgcgatttgc tggtgaccca atgcgaccag 1860
   atgctccacg cccagtcgcg taccgtcttc atgggagaaa ataatactgt tgatgggtgt 1920
   ctggtcagag acatcaagaa ataacgccgg aacattagtg caggcagctt ccacagcaat 1980
   ggcatcctgg tcatccagcg gatagttaat gatcagccca ctgacgcgtt gcgcgagaag 2040
   attgtgcacc gccgctttac aggcttcgac gccgcttcgt tctaccatcg acaccaccac 2100
   gctggcaccc agttgatcgg cgcgagattt aatcgccgcg acaatttgcg acggcgcgtg 2160
   cagggccaga ctggaggtgg caacgccaat cagcaacgac tgtttgcccg ccagttgttg 2220
   tgccacgcgg ttgggaatgt aattcagctc cgccatcgcc gcttccactt tttcccgcgt 2280
   tttcgcagaa acgtggctgg cctggttcac cacgcgggaa acggtctgat aagagacacc 2340
   ggcatactct gcgacatcgt ataacgttac tggtttcatc aaaatcgtct ccctccgttt 2400
   gaatatttga ttgatcgtaa ccagatgaag cactctttcc actatcccta cagtgttatg 2460
   gcttgaacaa tcacgaaaca ataattggta cgtacgatct ttcagccgac tcaaacatca 2520
   aatcttacaa atgtagtctt tgaaagtatt acatatgtaa gatttaaatg caaccgtttt 2580
   ttcggaagga aatgatgacc tcgtttccac cggaattagc ttggtaccaa aggaggtaag 2640
   gatcactaga aaatttttta aaaaatctct tgacattgga agggagatat gttattataa 2700
   gaattgcgga attgtgagcg gataacaatt cccatataga ttaactaata aggaggacaa 2760
   acatgggttc tatcatcaga cttcagggtg gtttaggcaa tcaactgttt caatttagct 2820
   ttggttatgc tctttcaaaa atcaatggca caccgctgta ctttgatatt tcacattacg 2880
   cagaaaacga tgatcatggc ggatatagac tgaataacct tcaaatcccg gaagaatatc 2940
   ttcagtacta cacaccgaaa atcaacaaca tctacaaact gcttgtcaga ggcagccgct 3000
   tatatccgga tatttttctg tttcttggct tttgcaacga atttcatgcg tatggctatg 3060
   attttgaata catcgcgcaa aaatggaaaa gcaaaaaata catcggatac tggcagtctg 3120
   aacatttctt tcataaacat attctggatc tgaaagaatt tttcatcccg aaaaacgtta 3180
   gcgaacaagc taacttactg gcagcgaaaa tcttagaatc tcagtcaagc ctgtcaattc 3240
   atatcagacg cggcgattac atcaaaaaca aaacagccac actgacacat ggagtttgct 3300
   ctcttgaata ctacaagaaa gcactgaaca aaatcagaga tttagccatg attcgcgatg 3360
   tgtttatctt ttctgatgat attttctggt gtaaagaaaa catcgaaaca cttctgagca 3420
   aaaaatacaa catctactac tcagaagatt taagccaaga agaagattta tggctgatgt 3480
   ctctggctaa tcatcatatt atcgccaact cttcattttc atggtggggc gcatatcttg 3540
   gaagctctgc gagccagatt gttatctatc cgacaccgtg gtatgatatt acaccgaaaa 3600
   acacatacat tcctatcgtg aaccattgga tcaacgtgga caaacattcc tcctgctaag 3660
   gagaaaagct agcgattaac taataaggag gaactgaaat gtcaaaagtg gcgcttatta 3720
   caggcgtcac aggacaagat ggcagctatt tagctgaatt tctgcttgaa aaaggatatg 3780
   aagtgcatgg cattaaaaga cgcgcatcat catttaacac agaaagagtc gatcatatct 3840
   atcaagatcc gcatacatgc aacccgaaat ttcatctgca ttatggcgat ctgagcgata 3900
   catctaacct gacaagaatt ttacgcgaag tccagccgga tgaagtttat aacctgggcg 3960
   cgatgtctca tgtcgctgtt tcatttgaaa gcccggaata tacagccgat gtcgatgcaa 4020
   tgggaacact tagattactg gaagccattc gctttttagg cctggaaaag aaaacacgct 4080
   tttatcaggc atctacatca gaactgtatg gacttgttca agaaatcccg cagaaagaaa 4140
   caacaccgtt ttatccgaga agcccgtatg cggtcgctaa actttacgcg tactggatca 4200
   cagttaatta tcgcgaatct tatggaatgt atgcttgcaa tggcatctta tttaaccatg 4260
   aatcaccgag acgcggagaa acatttgtta caagaaaaat cacacgcgcc attgcaaata 4320
   tcgcccaagg actggaaagc tgtctttatt taggcaacat ggattctctt agagattggg 4380
   gccatgcaaa agattacgtt aaaatgcaat ggatgatgtt acaacaggaa cagccggaag 4440
   attttgtgat cgccacagga gtgcaatatt cagtcagaca gtttgttgaa atggcagcgg 4500
   ctcagcttgg cattaaactg cgctttgaag gcacaggagt ggaagaaaaa ggaatcgttg 4560
   tgagcgttac aggccatgat gcgccgggag tgaaaccggg cgatgtgatt atcgccgtcg 4620
   atccgagata ttttcgcccg gcagaagtcg aaacactttt aggagatccg acaaaagcgc 4680
   atgaaaaact gggctggaaa ccggaaatta cactgagaga aatggttagc gaaatggtgg 4740
   ctaacgattt agaagccgcg aaaaaacatt ctctgcttaa atcacatggc tatgatgtgg 4800
   cgatcgctct ggaatcttaa aaaggaggaa ctactatgag caaacaaaga gtttttatcg 4860
   ctggccatcg cggaatggtg ggctctgcca ttagacgcca attagaacag agaggagatg 4920
   tggaacttgt cttaagaaca cgcgatgaac tgaatctgct tgattcaaga gctgtccatg 4980
   atttctttgc cagcgaacgc atcgatcagg tttatcttgc agcggctaaa gttggcggaa 5040
   ttgtggcaaa taacacatat ccggcggatt ttatctacca aaacatgatg atcgaatcta 5100
   acattatcca tgccgcacat cagaatgatg ttaacaaact gctgtttctg ggatcaagct 5160
   gcatctatcc gaaacttgca aaacaaccga tggcggaatc agaactttta cagggcacac 5220
   tggaaccgac aaatgaaccg tatgcaattg cgaaaatcgc gggaatcaaa ctttgtgaat 5280
   cttacaacag acaatacggc agagattatc gctcagtgat gccgacaaac ttatatggac 5340
   cgcatgataa ttttcatccg tctaactcac atgtcattcc ggcactgctt agacgctttc 5400
   atgaagctac agcccagaac gcgccggatg ttgtggtctg gggctcagga acaccgatgc 5460
   gcgaatttct gcatgttgat gatatggcgg ctgccagcat ccatgtgatg gaacttgctc 5520
   atgaagtctg gttagaaaat acacaaccga tgcttagcca tattaacgtc ggcacaggag 5580
   ttgattgcac aatcagagaa ttagctcaga caattgccaa agttgtggga tataaaggcc 5640
   gcgtcgtttt tgatgcctct aaaccggatg gcacaccgag aaaattactg gatgtgacac 5700
   gcctgcatca acttggatgg tatcatgaaa ttagcttaga agcaggcctg gcgtctacat 5760
   accaatggtt tttagaaaac caggatagat ttcgcggcgg atcataaagt gatagcggta 5820
   ccattatagg taagagagga atgtacacat gtactattta aaaaacacaa acttttggat 5880
   gttcggttta ttctttttct tttacttttt tatcatggga gcctacttcc cgtttttccc 5940
   gatttggcta catgacatca accatatcag caaaagtgat acgggtatta tttttgccgc 6000
   tatttctctg ttctcgctat tattccaacc gctgtttggt ctgctttctg acaaactcgg 6060
   gctgcgcaaa tacctgctgt ggattattac cggcatgtta gtgatgtttg cgccgttctt 6120
   tatttttatc ttcgggccac tgttacaata caacatttta gtaggatcga ttgttggtgg 6180
   tatttatcta ggcttttgtt ttaacgccgg tgcgccagca gtagaggcat ttattgagaa 6240
   agtcagccgt cgcagtaatt tcgaatttgg tcgcgcgcgg atgtttggct gtgttggctg 6300
   ggcgctgtgt gcctcgattg tcggcatcat gttcaccatc aataatcagt ttgttttctg 6360
   gctgggctct ggctgtgcac tcatcctcgc cgttttactc tttttcgcca aaacggatgc 6420
   gccctcttct gccacggttg ccaatgcggt aggtgccaac cattcggcat ttagccttaa 6480
   gctggcactg gaactgttca gacagccaaa actgtggttt ttgtcactgt atgttattgg 6540
   cgtttcctgc acctacgatg tttttgacca acagtttgct aatttcttta cttcgttctt 6600
   tgctaccggt gaacagggta cgcgggtatt tggctacgta acgacaatgg gcgaattact 6660
   taacgcctcg attatgttct ttgcgccact gatcattaat cgcatcggtg ggaaaaacgc 6720
   cctgctgctg gctggcacta ttatgtctgt acgtattatt ggctcatcgt tcgccacctc 6780
   agcgctggaa gtggttattc tgaaaacgct gcatatgttt gaagtaccgt tcctgctggt 6840
   gggctgcttt aaatatatta ccagccagtt tgaagtgcgt ttttcagcga cgatttatct 6900
   ggtctgtttc tgcttcttta agcaactggc gatgattttt atgtctgtac tggcgggcaa 6960
   tatgtatgaa agcatcggtt tccagggcgc ttatctggtg ctgggtctgg tggcgctggg 7020
   cttcacctta atttccgtgt tcacgcttag cggccccggc ccgctttccc tgctgcgtcg 7080
   tcaggtgaat gaagtcgctt aaggatccat gtctagagtc gacgtccccg gggcagcccg 7140
   cctaatgagc gggctttttt cacgtcccag gcatcaaata aaacgaaagg ctcagtcgaa 7200
   agactgggcc tttcgtttta tctgttgttt gtcggtgaac gctctctact agagtcacac 7260
   tggctcacct tcgggtgggc ctttctgcgt ttataccggg ggcagcccgc ctaatgagcg 7320
   ggcttttttc acgtcacgcg tccatggaga tctttgtctg caactgaaaa gtttatacct 7380
   tacctggaac aaatggttga aacatacgag gctaatatcg gcttattagg aatagtccct 7440
   gtactaataa aatcaggtgg atcagttgat cagtatattt tggacgaagc tcggaaagaa 7500
   tttggagatg acttgcttaa ttccacaatt aaattaaggg aaagaataaa gcgatttgat 7560
   gttcaaggaa tcacggaaga agatactcat gataaagaag ctctaaaact attcaataac 7620
   cttacaatgg aattgatcga aagggtggaa ggttaatggt acgaaaatta ggggatctac 7680
   ctagaaagcc acaaggcgat aggtcaagct taaagaaccc ttacatggat cttacagatt 7740
   ctgaaagtaa agaaacaaca gaggttaaac aaacagaacc aaaaagaaaa aaagcattgt 7800
   tgaaaacaat gaaagttgat gtttcaatcc ataataagat taaatcgctg cacgaaattc 7860
   tggcagcatc cgaagggaat tcatattact tagaggatac tattgagaga gctattgata 7920
   agatggttga gacattacct gagagccaaa aaacttttta tgaatatgaa ttaaaaaaaa 7980
   gaaccaacaa aggctgagac agactccaaa cgagtctgtt tttttaaaaa aaatattagg 8040
   agcattgaat atatattaga gaattaagaa agacatggga ataaaaatat tttaaatcca 8100
   gtaaaaatat gataagatta tttcagaata tgaagaactc tgtttgtttt tgatgaaaaa 8160
   acaaacaaaa aaaatccacc taacggaatc tcaatttaac taacagcggc caaactgaga 8220
   agttaaattt gagaagggga aaaggcggat ttatacttgt atttaactat ctccatttta 8280
   acattttatt aaaccccata caagtgaaaa tcctctttta cactgttcct ttaggtgatc 8340
   gcggagggac attatgagtg aagtaaacct aaaaggaaat acagatgaat tagtgtatta 8400
   tcgacagcaa accactggaa ataaaatcgc caggaagaga atcaaaaaag ggaaagaaga 8460
   agtttattat gttgctgaaa cggaagagaa gatatggaca gaagagcaaa taaaaaactt 8520
   ttctttagac aaatttggta cgcatatacc ttacatagaa ggtcattata caatcttaaa 8580
   taattacttc tttgattttt ggggctattt tttaggtgct gaaggaattg cgctctatgc 8640
   tcacctaact cgttatgcat acggcagcaa agacttttgc tttcctagtc tacaaacaat 8700
   cgctaaaaaa atggacaaga ctcctgttac agttagaggc tacttgaaac tgcttgaaag 8760
   gtacggtttt atttggaagg taaacgtccg taataaaacc aaggataaca cagaggaatc 8820
   cccgattttt aagattagac gtaaggttcc tttgctttca gaagaacttt taaatggaaa 8880
   ccctaatatt gaaattccag atgacgagga agcacatgta aagaaggctt taaaaaagga 8940
   aaaagagggt cttccaaagg ttttgaaaaa agagcacgat gaatttgtta aaaaaatgat 9000
   ggatgagtca gaaacaatta atattccaga ggccttacaa tatgacacaa tgtatgaaga 9060
   tatactcagt aaaggagaaa ttcgaaaaga aatcaaaaaa caaataccta atcctacaac 9120
   atcttttgag agtatatcaa tgacaactga agaggaaaaa gtcgacagta ctttaaaaag 9180
   cgaaatgcaa aatcgtgtct ctaagccttc ttttgatacc tggtttaaaa acactaagat 9240
   caaaattgaa aataaaaatt gtttattact tgtaccgagt gaatttgcat ttgaatggat 9300
   taagaaaaga tatttagaaa caattaaaac agtccttgaa gaagctggat atgttttcga 9360
   aaaaatcgaa ctaagaaaag tgcaataaac tgctgaagta tttcagcagt tttttttatt 9420
   tagaaatagt gaaaaaaata taatcaggga ggtatcaata tttaatgagt actgatttaa 9480
   atttatttag actggaatta ataattaaca cgtagactaa ttaaaattta atgagggata 9540
   aagaggatac aaaaatatta atttcaatcc ctattaaatt ttaacaaggg ggggattaaa 9600
   atttaattag aggtttatcc acaagaaaag accctaataa aatttttact agggttataa 9660
   cactgattaa tttcttaatg ggggagggat taaaatttaa tgacaaagaa aacaatcttt 9720
   taagaaaagc ttttaaaaga taataataaa aagagctttg cgattaagca aaactcttta 9780
   ctttttcatt gacattatca aattcatcga tttcaaattg ttgttgtatc ataaagttaa 9840
   ttctgttttg cacaaccttt tcaggaatat aaaacacatc tgaggcttgt tttataaact 9900
   cagggtcgct aaagtcaatg taacgtagca tatgatatgg tatagcttcc acccaagtta 9960
   gcctttctgc ttcttctgaa tgtttttcat atacttccat gggtatctct aaatgatttt 10020
   cctcatgtag caaggtatga gcaaaaagtt tatggaattg atagttcctc tctttttctt 10080
   caactttttt atctaaaaca aacactttaa catctgagtc aatgtaagca taagatgttt 10140
   ttccagtcat aatttcaatc ccaaatcttt tagacagaaa ttctggacgt aaatcttttg 10200
   gtgaaagaat ttttttatgt agcaatatat ccgatacagc accttctaaa agcgttggtg 10260
   aatagggcat tttacctatc tcctctcatt ttgtggaata aaaatagtca tattcgtcca 10320
   tctacctatc ctattatcga acagttgaac tttttaatca aggatcagtc ctttttttca 10380
   ttattcttaa actgtgctct taactttaac aactcgattt gtttttccag atctcgaggg 10440
   taactagcct cgccgatccc gcaagaggcc cggcagtcag gtggcacttt tcggggaaat 10500
   gtgcgcggaa cccctatttg tttatttttc taaatacatt caaatatgta tccgctcatg 10560
   agacaataac cctgataaat gcttcaataa tattgaaaaa ggaagagtat gagtattcaa 10620
   catttccgtg tcgcccttat tccctttttt gcggcatttt gccttcctgt ttttgctcac 10680
   ccagaaacgc tggtgaaagt aaaagatgct gaagatcagt tgggtgcacg agtgggttac 10740
   atcgaactgg atctcaacag cggtaagatc cttgagagtt ttcgccccga agaacgtttt 10800
   ccaatgatga gcacttttaa agttctgcta tgtggcgcgg tattatcccg tattgacgcc 10860
   gggcaagagc aactcggtcg ccgcatacac tattctcaga atgacttggt tgagtactca 10920
   ccagtcacag aaaagcatct tacggatggc atgacagtaa gagaattatg cagtgctgcc 10980
   ataaccatga gtgataacac tgcggccaac ttacttctga caacgatcgg aggaccgaag 11040
   gagctaaccg cttttttgca caacatgggg gatcatgtaa ctcgccttga tcgttgggaa 11100
   ccggagctga atgaagccat accaaacgac gagcgtgaca ccacgatgcc tgtagcaatg 11160
   gcaacaacgt tgcgcaaact attaactggc gaactactta ctctagcttc ccggcaacaa 11220
   ttaatagact ggatggaggc ggataaagtt gcaggaccac ttctgcgctc ggcccttccg 11280
   gctggctggt ttattgctga taaatctgga gccggtgagc gtgggtctcg cggtatcatt 11340
   gcagcactgg ggccagatgg taagccctcc cgtatcgtag ttatctacac gacggggagt 11400
   caggcaacta tggatgaacg aaatagacag atcgctgaga taggtgcctc actgattaag 11460
   cattggtaac tgtcagacca agtttactca tatatacttt agattgattt aaaacttcat 11520
   ttttaattta aaaggatcta ggtgaagatc ctttttgata atctcatgac caaaatccct 11580
   taacgtgagt tttcgttcca ctgagcgtca gaccccgtag aaaagatcaa aggatcttct 11640
   tgagatcctt tttttctgcg cgtaatctgc tgcttgcaaa caaaaaaacc accgctacca 11700
   gcggtggttt gtttgccgga tcaagagcta ccaactcttt ttccgaaggt aactggcttc 11760
   agcagagcgc agataccaaa tactgtcctt ctagtgtagc cgtagttagg ccaccacttc 11820
   aagaactctg tagcaccgcc tacatacctc gctctgctaa tcctgttacc agtggctgct 11880
   gccagtggcg ataagtcgtg tcttaccggg ttggactcaa gacgatagtt accggataag 11940
   gcgcagcggt cgggctgaac ggggggttcg tgcacacagc ccagcttgga gcgaacgacc 12000
   tacaccgaac tgagatacct acagcgtgag ctatgagaaa gcgccacgct tcccgaaggg 12060
   agaaaggcgg acaggtatcc ggtaagcggc agggtcggaa caggagagcg cacgagggag 12120
   cttccagggg gaaacgcctg gtatctttat agtcctgtcg ggtttcgcca cctctgactt 12180
   gagcgtcgat ttttgtgatg ctcgtcaggg gggcggagcc tatggaaaaa cgccagcaac 12240
   gcggcctttt tacggttcct ggccttttgc tggccttttg ctcacatgtt ctttcctgcg 12300
   ttatcccctg attctgtgga taaccgtatt accgcctttg agtgagctga taccgctcgc 12360
   cgcagccgaa cgaccgagcg cagcgagtca gtgagcgagg aagcggaaga gcgcccaata 12420
   cgcatgc 12427

<210> 3
<211> 12330
<212> DNA
<213> Artificial Sequence

<220>
<223> integration vector

<400> 3
ttctcatgtt tgacagctta tcatcgataa gctttaatgc ggtagtttat cacagttaaa 60

ttgctaacgc agtcaggcac cgtgtatgaa atctaacaat gcgctcatcg tcatcctcgg 120

caccgtcacc ctggatgctg taggcatagg cttggttatg ccggtactgc cgggcctctt 180

gcgggatatc gtccattccg acagcatcgc cagtcactat ggcgtgctgc tagcgctata 240

tgcgttgatg caatttctat gcgcacccgt tctcggagca ctgtccgacc gctttggccg 300

ccgcccagtc ctgctcgctt cgctacttgg agccactatc gactacgcga tcatggcgac 360

cacacccgtc ctgtggatcc tctacgccgg acgcatcgtg gccggcatca ccggcgccac 420

aggtgcggtt gctggcgcct atatcgccga catcaccgat ggggaagatc gggctcgcca 480

cttcgggctc atgagcgctt gtttcggcgt gggtatggtg gcaggccccg tggccggggg 540

actgttgggc gccatctcct tgcatgcacc attccttgcg gcggcggtgc tcaacggcct 600

caacctacta ctgggctgct tcctaatgca ggagtcgcat aagggagagc gtcgaccgat 660

gcccttgaga gccttcaacc cagtcagctc cttccggtgg gcgcggggca tgactatcgt 720

cgccgcactt atgactgtct tctttatcat gcaactcgta ggacaggtgc cggcagcgct 780

ctgggtcatt ttcggcgagg accgctttcg ctggagcgcg acgatgatcg gcctgtcgct 840

tgcggtattc ggaatcttgc acgccctcgc tcaagccttc gtcactggtc ccgccaccaa 900

acgtttcggc gagaagcagg ccattatcgc cggcatggcg gccgacgcgc tgggctacgt 960

cttgctggcg ttcgcgacgc gaggctggat ggccttcccc attatgattc ttctcgcttc 1020

cggcggcatc gggatgcccg cgttgcaggc catgctgtcc aggcaggtag atgacgacca 1080

tcagggacag cttcaaggat cgctcgcggc tcttaccagc ctaacttcga tcactggacc 1140

gctgatcgtc acggcgattt atgccgcctc ggcgagcaca tggaacgggt tggcatggat 1200

tgtaggcgcc gccctatacc ttgtctgcct ccccgcgttg cgtcgcggtg catggagccg 1260

ggccacctcg acctgaatgg aagccggcgg cacctcgcta acggattcac cactccaaga 1320

attggagcca atcaattctt gcggagaact gtgaatgcgc aaaccaaccc ttggcagaac 1380

atatccatcg cgtccgccat ctccagcagc cgcacgcggc gcatctcggg cagcgttggg 1440

tcctggccac gggtgcgcat gatcgtgctc ctgtcgttga ggacccggct aggctggcgg 1500

ggttgcctta ctggttagca gaatgaatca ccgatacgcg agcgaacgtg aagcgactgc 1560

tgctgcaaaa cgtctgcgac ctgagcaaca acatgaatgg tcttcggttt ccgtgtttcg 1620

taaagtctgg aaacgcggaa gtcagcgccc tgcaccatta tgttccggat ctgcatcgca 1680

ggatgctgct ggctaccctg tggaacacct acatctgtat taacgaagcg ctggcattga 1740

ccctgagtga tttttctctg gtcccgccgc atccataccg ccagttgttt accctcacaa 1800

cgttccagta accgggcatg ttcatcatca gtaacccgta tcgtgagcat cctctctcgt 1860

ttcatcggta tcattacccc catgaacaga aatccccctt acacggaggc atcagtgacc 1920

aaacaggaaa aaaccgccct taacatggcc cgctttatca gaagccagac attaacgctt 1980

ctggagaaac tcaacgagct ggacgcggat gaacaggcag acatctgtga atcgcttcac 2040

gaccacgctg atgagcttta ccgcagctgc ctcgcgcgtt tcggtgatga cggtgaaaac 2100

ctctgacaca tgcagctccc ggagacggtc acagcttgtc tgtaagcgga tgccgggagc 2160

agacaagccc gtcagggcgc gtcagcgggt gttggcgggt gtcggggcgc agccatgacc 2220

cagtcacgta gcgatagcgg agtgtatact ggcttaacta tgcggcatca gagcagattg 2280

tactgagagt gcaccatatg acccgacatc cggcgttctc atggcggtgc ttgccgccag 2340

cggtattccg tatgtcaagt ggctgcggtt tatggtgccg cttgctctga tttggttctt 2400

gatcgggctt gtctttatcg tgatcggagt catgatcaat tgggggccgt tttaacgatt 2460

gctgcccgcc ggcttgtacg gcgggctttt gagttattca ttgcagaagc gcaggctgtt 2520

attgtaacat gtaagccata agccattcgt aaaagtgcgg gaggaaggtc atgaataatc 2580

tgcgtaatag actttcaggc gtgaatggga aaaataagag agtaaaagaa aaagaacaaa 2640

aaatctggtc ggagattggg atgatagcgg gagcatttgc gctgcttgat gtgatcatcc 2700

gcggcattat gtttgaattt ccgtttaaag aatgggctgc aagccttgtg tttttgttca 2760

tcattatctt atattactgc atcagggctg cggcatccgg aatgctcatg ccgagaatag 2820

acaccaaaga agaactgcaa aaacgggtga agcagcagcg aatagaatca attgcggtcg 2880

cctttgcggt agtggtgctt acgatgtacg acagggggat tccccataca ttcttcgctt 2940

ggctgaaaat gattcttctt tttatcgtct gcggcggcgt tctgtttctg cttcggtatg 3000

tgattgtgaa gctggcttac agaagagcgg taaaagaaga aataaaaaag aaatcatctt 3060

ttttgtttgg aaagcgaggg aagcgttcac agtttcgggc agcttttttt ataggaacat 3120

tgatttgtat tcactctgcc aagttgtttt gatagagtga ttgtgataat tttaaatgta 3180

agcgttaaca aaattctcca gtcttcacat cggtttgaaa ggaggaagcg gaagaatgaa 3240

gtaagaggga tttttgactc cgaagtaagt cttcaaaaaa tcaaataagg agtgtcaaga 3300

ccagtgaatt cgagctcggt acctaccgtt cgtataatgt atgctatacg aagttatgat 3360

aaaaaattta gaagccaatg aaatctataa ataaactaaa ttaagtttat ttaattaaca 3420

actatggata taaaataggt actaatcaaa atagtgagga ggatatattt gaatacatac 3480

gaacaaatta ataaagtgaa aaaaatactt cggaaacatt taaaaaataa ccttattggt 3540

acttacatgt ttggatcagg agttgagagt ggactaaaac caaatagtga tcttgacttt 3600

ttagtcgtcg tatctgaacc attgacagat caaagtaaag aaatacttat acaaaaaatt 3660

agacctattt caaaaaaaat aggagataaa agcaacttac gatatattga attaacaatt 3720

attattcagc aagaaatggt accgtggaat catcctccca aacaagaatt tatttatgga 3780

gaatggttac aagagcttta tgaacaagga tacattcctc agaaggaatt aaattcagat 3840

ttaaccataa tgctttacca agcaaaacga aaaaataaaa gaatatacgg aaattatgac 3900

ttagaggaat tactacctga tattccattt tctgatgtga gaagagccat tatggattcg 3960

tcagaggaat taatagataa ttatcaggat gatgaaacca actctatatt aactttatgc 4020

cgtatgattt taactatgga cacgggtaaa atcataccaa aagatattgc gggaaatgca 4080

gtggctgaat cttctccatt agaacatagg gagagaattt tgttagcagt tcgtagttat 4140

cttggagaga atattgaatg gactaatgaa aatgtaaatt taactataaa ctatttaaat 4200

aacagattaa aaaaattata aaaaaattga aaaaatggtg gaaacacttt tttcaatttt 4260

tttgttttat tatttaatat ttgggaaata ttcattctaa tataacttcg tataatgtat 4320

gctatacgaa cggtaggatc ctctagagtc gacctgcagg cattttacat ttttagaaat 4380

gggcgtgaaa aaaagcgcgc gattatgtaa aatataaaga ttaactaata aggaggacaa 4440

acatgggttc tatcatcaga cttcagggtg gtttaggcaa tcaactgttt caatttagct 4500

ttggttatgc tctttcaaaa atcaatggca caccgctgta ctttgatatt tcacattacg 4560

cagaaaacga tgatcatggc ggatatagac tgaataacct tcaaatcccg gaagaatatc 4620

ttcagtacta cacaccgaaa atcaacaaca tctacaaact gcttgtcaga ggcagccgct 4680

tatatccgga tatttttctg tttcttggct tttgcaacga atttcatgcg tatggctatg 4740

attttgaata catcgcgcaa aaatggaaaa gcaaaaaata catcggatac tggcagtctg 4800

aacatttctt tcataaacat attctggatc tgaaagaatt tttcatcccg aaaaacgtta 4860

gcgaacaagc taacttactg gcagcgaaaa tcttagaatc tcagtcaagc ctgtcaattc 4920

atatcagacg cggcgattac atcaaaaaca aaacagccac actgacacat ggagtttgct 4980

ctcttgaata ctacaagaaa gcactgaaca aaatcagaga tttagccatg attcgcgatg 5040

tgtttatctt ttctgatgat attttctggt gtaaagaaaa catcgaaaca cttctgagca 5100

aaaaatacaa catctactac tcagaagatt taagccaaga agaagattta tggctgatgt 5160

ctctggctaa tcatcatatt atcgccaact cttcattttc atggtggggc gcatatcttg 5220

gaagctctgc gagccagatt gttatctatc cgacaccgtg gtatgatatt acaccgaaaa 5280

acacatacat tcctatcgtg aaccattgga tcaacgtgga caaacattcc tcctgctaaa 5340

gtgatagcgg taccattata ggtaagagag gaatgtacac atgtactatt taaaaaacac 5400

aaacttttgg atgttcggtt tattcttttt cttttacttt tttatcatgg gagcctactt 5460

cccgtttttc ccgatttggc tacatgacat caaccatatc agcaaaagtg atacgggtat 5520

tatttttgcc gctatttctc tgttctcgct attattccaa ccgctgtttg gtctgctttc 5580

tgacaaactc gggctgcgca aatacctgct gtggattatt accggcatgt tagtgatgtt 5640

tgcgccgttc tttattttta tcttcgggcc actgttacaa tacaacattt tagtaggatc 5700

gattgttggt ggtatttatc taggcttttg ttttaacgcc ggtgcgccag cagtagaggc 5760

atttattgag aaagtcagcc gtcgcagtaa tttcgaattt ggtcgcgcgc ggatgtttgg 5820

ctgtgttggc tgggcgctgt gtgcctcgat tgtcggcatc atgttcacca tcaataatca 5880

gtttgttttc tggctgggct ctggctgtgc actcatcctc gccgttttac tctttttcgc 5940

caaaacggat gcgccctctt ctgccacggt tgccaatgcg gtaggtgcca accattcggc 6000

atttagcctt aagctggcac tggaactgtt cagacagcca aaactgtggt ttttgtcact 6060

gtatgttatt ggcgtttcct gcacctacga tgtttttgac caacagtttg ctaatttctt 6120

tacttcgttc tttgctaccg gtgaacaggg tacgcgggta tttggctacg taacgacaat 6180

gggcgaatta cttaacgcct cgattatgtt ctttgcgcca ctgatcatta atcgcatcgg 6240

tgggaaaaac gccctgctgc tggctggcac tattatgtct gtacgtatta ttggctcatc 6300

gttcgccacc tcagcgctgg aagtggttat tctgaaaacg ctgcatatgt ttgaagtacc 6360

gttcctgctg gtgggctgct ttaaatatat taccagccag tttgaagtgc gtttttcagc 6420

gacgatttat ctggtctgtt tctgcttctt taagcaactg gcgatgattt ttatgtctgt 6480

actggcgggc aatatgtatg aaagcatcgg tttccagggc gcttatctgg tgctgggtct 6540

ggtggcgctg ggcttcacct taatttccgt gttcacgctt agcggccccg gcccgctttc 6600

cctgctgcgt cgtcaggtga atgaagtcgc ttaaagtcaa tgtatgaatg gatacgggat 6660

atgaatcaat aagtacgtga aagagaaaag caacccagat atgataggga acttttctct 6720

ttcttgtttt acattgaatc tttacaatcc tattgatata atctaagcta gtgtattttg 6780

cgtttaatag tggagaaaag ctagcgatta actaataagg aggaactgaa atgtcaaaag 6840

tggcgcttat tacaggcgtc acaggacaag atggcagcta tttagctgaa tttctgcttg 6900

aaaaaggata tgaagtgcat ggcattaaaa gacgcgcatc atcatttaac acagaaagag 6960

tcgatcatat ctatcaagat ccgcatacat gcaacccgaa atttcatctg cattatggcg 7020

atctgagcga tacatctaac ctgacaagaa ttttacgcga agtccagccg gatgaagttt 7080

ataacctggg cgcgatgtct catgtcgctg tttcatttga aagcccggaa tatacagccg 7140

atgtcgatgc aatgggaaca cttagattac tggaagccat tcgcttttta ggcctggaaa 7200

agaaaacacg cttttatcag gcatctacat cagaactgta tggacttgtt caagaaatcc 7260

cgcagaaaga aacaacaccg ttttatccga gaagcccgta tgcggtcgct aaactttacg 7320

cgtactggat cacagttaat tatcgcgaat cttatggaat gtatgcttgc aatggcatct 7380

tatttaacca tgaatcaccg agacgcggag aaacatttgt tacaagaaaa atcacacgcg 7440

ccattgcaaa tatcgcccaa ggactggaaa gctgtcttta tttaggcaac atggattctc 7500

ttagagattg gggccatgca aaagattacg ttaaaatgca atggatgatg ttacaacagg 7560

aacagccgga agattttgtg atcgccacag gagtgcaata ttcagtcaga cagtttgttg 7620

aaatggcagc ggctcagctt ggcattaaac tgcgctttga aggcacagga gtggaagaaa 7680

aaggaatcgt tgtgagcgtt acaggccatg atgcgccggg agtgaaaccg ggcgatgtga 7740

ttatcgccgt cgatccgaga tattttcgcc cggcagaagt cgaaacactt ttaggagatc 7800

cgacaaaagc gcatgaaaaa ctgggctgga aaccggaaat tacactgaga gaaatggtta 7860

gcgaaatggt ggctaacgat ttagaagccg cgaaaaaaca ttctctgctt aaatcacatg 7920

gctatgatgt ggcgatcgct ctggaatctt aaaaaggagg aactactatg agcaaacaaa 7980

gagtttttat cgctggccat cgcggaatgg tgggctctgc cattagacgc caattagaac 8040

agagaggaga tgtggaactt gtcttaagaa cacgcgatga actgaatctg cttgattcaa 8100

gagctgtcca tgatttcttt gccagcgaac gcatcgatca ggtttatctt gcagcggcta 8160

aagttggcgg aattgtggca aataacacat atccggcgga ttttatctac caaaacatga 8220

tgatcgaatc taacattatc catgccgcac atcagaatga tgttaacaaa ctgctgtttc 8280

tgggatcaag ctgcatctat ccgaaacttg caaaacaacc gatggcggaa tcagaacttt 8340

tacagggcac actggaaccg acaaatgaac cgtatgcaat tgcgaaaatc gcgggaatca 8400

aactttgtga atcttacaac agacaatacg gcagagatta tcgctcagtg atgccgacaa 8460

acttatatgg accgcatgat aattttcatc cgtctaactc acatgtcatt ccggcactgc 8520

ttagacgctt tcatgaagct acagcccaga acgcgccgga tgttgtggtc tggggctcag 8580

gaacaccgat gcgcgaattt ctgcatgttg atgatatggc ggctgccagc atccatgtga 8640

tggaacttgc tcatgaagtc tggttagaaa atacacaacc gatgcttagc catattaacg 8700

tcggcacagg agttgattgc acaatcagag aattagctca gacaattgcc aaagttgtgg 8760

gatataaagg ccgcgtcgtt tttgatgcct ctaaaccgga tggcacaccg agaaaattac 8820

tggatgtgac acgcctgcat caacttggat ggtatcatga aattagctta gaagcaggcc 8880

tggcgtctac ataccaatgg tttttagaaa accaggatag atttcgcggc ggatcataag 8940

gatccatgtc tagagtcgac gtccccgggg cagcccgcct aatgagcggg cttttttcac 9000

gtcccaggca tcaaataaaa cgaaaggctc agtcgaaaga ctgggccttt cgttttatct 9060

gttgtttgtc ggtgaacgct ctctactaga gtcacactgg ctcaccttcg ggtgggcctt 9120

tctgcgttta tagaattcat attacttaga ggatactatt gagagagcta ttgataagat 9180

ggttgagaca ttacctgaga gccaaaaaac tttttatgaa tatgaattaa aaaaaagaac 9240

caacaaaggc tgagacagac tccgggcaag gctagacggg acttaccgaa agaaaccatc 9300

aatgatggtt tcttttttgt tcataaatca gacaaaactt ttctcttgca aaagtttgtg 9360

aagtgttgca caatataaat gtgaaatact tcacaaacaa aaagacatca aagagaaaca 9420

taccctggaa ggatgattaa tgatgaacaa acatgtaaat aaagtagctt taatcggagc 9480

gggttttgtt ggaagcagtt atgcatttgc gttaattaac caaggaatca cagatgagct 9540

tgtggtcatt gatgtaaata aagaaaaagc aatgggcgat gtgatggatt taaaccacgg 9600

aaaggcgttt gcgccacaac cggtcaaaac atcttacgga acatatgaag actgcaagga 9660

tgctgatatt gtctgcattt gcgccggagc aaaccaaaaa cctggtgaga cacgccttga 9720

attagtagaa aagaacttga agattttcaa aggcatcgtt agtgaagtca tggcgagcgg 9780

atttgacggc attttcttag tcgcgacaaa tccggttgat atcctgactt acgcaacatg 9840

gaaattcagc ggcctgccaa aagagcgggt gattggaagc ggcacaacac ttgattctgc 9900

gagattccgt ttcatgctga gcgaatactt tggcgcagcg cctcaaaacg tacacgcgca 9960

tattatcgga gagcacggcg acacagagct tcctgtttgg agccacgcga atgtcggcgg 10020

tgtgccggtc agtgaactcg ttgagaaaaa cgatgcgtac aaacaagagg agctggacca 10080

aattgtagat gatgtgaaaa acgcagctta ccatatcatt gagaaaaaag gcgcgactta 10140

ttatggggtt gcgatgagtc ttgctcgcat tacaaaagcc attcttcata atgaaaacag 10200

catattaact gtcagcacat atttggacgg gcaatacggt gcagatgacg tgtacatcgg 10260

tgtcatatgc ggtgtgaaat accgcacaga tgcgtaagga gaaaataccg catcaggcgc 10320

tcttccgctt cctcgctcac tgactcgctg cgctcggtcg ttcggctgcg gcgagcggta 10380

tcagctcact caaaggcggt aatacggtta tccacagaat caggggataa cgcaggaaag 10440

aacatgtgag caaaaggcca gcaaaaggcc aggaaccgta aaaaggccgc gttgctggcg 10500

tttttccata ggctccgccc ccctgacgag catcacaaaa atcgacgctc aagtcagagg 10560

tggcgaaacc cgacaggact ataaagatac caggcgtttc cccctggaag ctccctcgtg 10620

cgctctcctg ttccgaccct gccgcttacc ggatacctgt ccgcctttct cccttcggga 10680

agcgtggcgc tttctcatag ctcacgctgt aggtatctca gttcggtgta ggtcgttcgc 10740

tccaagctgg gctgtgtgca cgaacccccc gttcagcccg accgctgcgc cttatccggt 10800

aactatcgtc ttgagtccaa cccggtaaga cacgacttat cgccactggc agcagccact 10860

ggtaacagga ttagcagagc gaggtatgta ggcggtgcta cagagttctt gaagtggtgg 10920

cctaactacg gctacactag aaggacagta tttggtatct gcgctctgct gaagccagtt 10980

accttcggaa aaagagttgg tagctcttga tccggcaaac aaaccaccgc tggtagcggt 11040

ggtttttttg tttgcaagca gcagattacg cgcagaaaaa aaggatctca agaagatcct 11100

ttgatctttt ctacggggtc tgacgctcag tggaacgaaa actcacgtta agggattttg 11160

gtcatgagat tatcaaaaag gatcttcacc tagatccttt taaattaaaa atgaagtttt 11220

aaatcaatct aaagtatata tgagtaaact tggtctgaca gttaccaatg cttaatcagt 11280

gaggcaccta tctcagcgat ctgtctattt cgttcatcca tagttgcctg actccccgtc 11340

gtgtagataa ctacgatacg ggagggctta ccatctggcc ccagtgctgc aatgataccg 11400

cgagacccac gctcaccggc tccagattta tcagcaataa accagccagc cggaagggcc 11460

gagcgcagaa gtggtcctgc aactttatcc gcctccatcc agtctattaa ttgttgccgg 11520

gaagctagag taagtagttc gccagttaat agtttgcgca acgttgttgc cattgctgca 11580

ggcatcgtgg tgtcacgctc gtcgtttggt atggcttcat tcagctccgg ttcccaacga 11640

tcaaggcgag ttacatgatc ccccatgttg tgcaaaaaag cggttagctc cttcggtcct 11700

ccgatcgttg tcagaagtaa gttggccgca gtgttatcac tcatggttat ggcagcactg 11760

cataattctc ttactgtcat gccatccgta agatgctttt ctgtgactgg tgagtactca 11820

accaagtcat tctgagaata gtgtatgcgg cgaccgagtt gctcttgccc ggcgtcaaca 11880

cgggataata ccgcgccaca tagcagaact ttaaaagtgc tcatcattgg aaaacgttct 11940

tcggggcgaa aactctcaag gatcttaccg ctgttgagat ccagttcgat gtaacccact 12000

cgtgcaccca actgatcttc agcatctttt actttcacca gcgtttctgg gtgagcaaaa 12060

acaggaaggc aaaatgccgc aaaaaaggga ataagggcga cacggaaatg ttgaatactc 12120

atactcttcc tttttcaata ttattgaagc atttatcagg gttattgtct catgagcgga 12180

tacatatttg aatgtattta gaaaaataaa caaatagggg ttccgcgcac atttccccga 12240

aaagtgccac ctgacgtcta agaaaccatt attatcatga cattaaccta taaaaatagg 12300

cgtatcacga ggccctttcg tcttcaagaa 12330

<210> 4
<211> 4254
<212> DNA
<213> Artificial Sequence

<220>
<223> integration cassette

<400> 4
gcaggctgtt attgtaacat gtaagccata agccattcgt aaaagtgcgg gaggaaggtc 60

atgaataatc tgcgtaatag actttcaggc gtgaatggga aaaataagag agtaaaagaa 120

aaagaacaaa aaatctggtc ggagattggg atgatagcgg gagcatttgc gctgcttgat 180

gtgatcatcc gcggcattat gtttgaattt ccgtttaaag aatgggctgc aagccttgtg 240
tttttgttca tcattatctt atattactgc atcagggctg cggcatccgg aatgctcatg 300
ccgagaatag acaccaaaga agaactgcaa aaacgggtga agcagcagcg aatagaatca 360
attgcggtcg cctttgcggt agtggtgctt acgatgtacg acagggggat tccccataca 420
ttcttcgctt ggctgaaaat gattcttctt tttatcgtct gcggcggcgt tctgtttctg 480
cttcggtatg tgattgtgaa gctggcttac agaagagcgg taaaagaaga aataaaaaag 540
aaatcatctt ttttgtttgg aaagcgaggg aagcgttcac agtttcgggc agcttttttt 600
ataggaacat tgatttgtat tcactctgcc aagttgtttt gatagagtga ttgtgataat 660
tttaaatgta agcgttaaca aaattctcca gtcttcacat cggtttgaaa ggaggaagcg 720
gaagaatgaa gtaagaggga tttttgactc cgaagtaagt cttcaaaaaa tcaaataagg 780
agtgtcaaga atgtttgcaa aacgattcaa aacctcttta ctgccgttat tcgctggatt 840
tttattgctg tttcatttgg ttctggcagg accggcggct gcgagtgctg aaacggcgaa 900
caaatcgaat gagcttacag caccgtcgat caaaagcgga accattcttc atgcatggcc 960
agtgaattcg agctcggtac ctaccgttcg tataatgtat gctatacgaa gttatgataa 1020
aaaatttaga agccaatgaa atctataaat aaactaaatt aagtttattt aattaacaac 1080
tatggatata aaataggtac taatcaaaat agtgaggagg atatatttga atacatacga 1140
acaaattaat aaagtgaaaa aaatacttcg gaaacattta aaaaataacc ttattggtac 1200
ttacatgttt ggatcaggag ttgagagtgg actaaaacca aatagtgatc ttgacttttt 1260
agtcgtcgta tctgaaccat tgacagatca aagtaaagaa atacttatac aaaaaattag 1320
acctatttca aaaaaaatag gagataaaag caacttacga tatattgaat taacaattat 1380
tattcagcaa gaaatggtac cgtggaatca tcctcccaaa caagaattta tttatggaga 1440
atggttacaa gagctttatg aacaaggata cattcctcag aaggaattaa attcagattt 1500
aaccataatg ctttaccaag caaaacgaaa aaataaaaga atatacggaa attatgactt 1560
agaggaatta ctacctgata ttccattttc tgatgtgaga agagccatta tggattcgtc 1620
agaggaatta atagataatt atcaggatga tgaaaccaac tctatattaa ctttatgccg 1680
tatgatttta actatggaca cgggtaaaat catacccaaa gatattgcgg gaaatgcagt 1740
ggctgaatct tctccattag aacataggga gagaattttg ttagcagttc gtagttatct 1800
tggagagaat attgaatgga ctaatgaaaa tgtaaattta actataaact atttaaataa 1860
cagattaaaa aaattataaa aaaattgaaa aaatggtgga aacacttttt tcaatttttt 1920
tgttttatta tttaatattt gggaaatatt cattctaata taacttcgta taatgtatgc 1980
tatacgaacg gtaggatcct ctagagtcga cctgcaggca ttttacattt ttagaaatgg 2040

gcgtgaaaaa aagcgcgcga ttatgtaaaa tataaagtga tagcggtacc attataggta 2100

agagaggaat gtacacatgt actatttaaa aaacacaaac ttttggatgt tcggtttatt 2160

ctttttcttt tactttttta tcatgggagc ctacttcccg tttttcccga tttggctaca 2220

tgacatcaac catatcagca aaagtgatac gggtattatt tttgccgcta tttctctgtt 2280

ctcgctatta ttccaaccgc tgtttggtct gctttctgac aaactcgggc tgcgcaaata 2340

cctgctgtgg attattaccg gcatgttagt gatgtttgcg ccgttcttta tttttatctt 2400

cgggccactg ttacaataca acattttagt aggatcgatt gttggtggta tttatctagg 2460

cttttgtttt aacgccggtg cgccagcagt agaggcattt attgagaaag tcagccgtcg 2520

cagtaatttc gaatttggtc gcgcgcggat gtttggctgt gttggctggg cgctgtgtgc 2580

ctcgattgtc ggcatcatgt tcaccatcaa taatcagttt gttttctggc tgggctctgg 2640

ctgtgcactc atcctcgccg ttttactctt tttcgccaaa acggatgcgc cctcttctgc 2700

cacggttgcc aatgcggtag gtgccaacca ttcggcattt agccttaagc tggcactgga 2760

actgttcaga cagccaaaac tgtggttttt gtcactgtat gttattggcg tttcctgcac 2820

ctacgatgtt tttgaccaac agtttgctaa tttctttact tcgttctttg ctaccggtga 2880

acagggtacg cgggtatttg gctacgtaac gacaatgggc gaattactta acgcctcgat 2940

tatgttcttt gcgccactga tcattaatcg catcggtggg aaaaacgccc tgctgctggc 3000

tggcactatt atgtctgtac gtattattgg ctcatcgttc gccacctcag cgctggaagt 3060

ggttattctg aaaacgctgc atatgtttga agtaccgttc ctgctggtgg gctgctttaa 3120

atatattacc agccagtttg aagtgcgttt ttcagcgacg atttatctgg tctgtttctg 3180

cttctttaag caactggcga tgatttttat gtctgtactg gcgggcaata tgtatgaaag 3240

catcggtttc cagggcgctt atctggtgct gggtctggtg gcgctgggct tcaccttaat 3300

ttccgtgttc acgcttagcg gccccggccc gctttccctg ctgcgtcgtc aggtgaatga 3360

agtcgcttaa gcaatcaatg tcggatgcca gcctggcttt gattacgtgc taaatggttt 3420

atataatgac tcgggcttaa gcggttctct tccccattga gggcaaggct agacgggact 3480

taccgaaaga aaccatcaat gatggtttct tttttgttca taaatcagac aaaacttttc 3540

tcttgcaaaa gtttgtgaag tgttgcacaa tataaatgtg aaatacttca caaacaaaaa 3600

gacatcaaag agaaacatac cctggaagga tgattaatga tgaacaaaca tgtaaataaa 3660

gtagctttaa tcggagcggg ttttgttgga agcagttatg catttgcgtt aattaaccaa 3720

ggaatcacag atgagcttgt ggtcattgat gtaaataaag aaaaagcaat gggcgatgtg 3780

atggatttaa accacggaaa ggcgtttgcg ccacaaccgg tcaaaacatc ttacggaaca 3840

tatgaagact gcaaggatgc tgatattgtc tgcatttgcg ccggagcaaa ccaaaaacct 3900

ggtgagacac gccttgaatt agtagaaaag aacttgaaga ttttcaaagg catcgttagt 3960

gaagtcatgg cgagcggatt tgacggcatt ttcttagtcg cgacaaatcc ggttgatatc 4020

ctgacttacg caacatggaa attcagcggc ctgccaaaag agcgggtgat tggaagcggc 4080

acaacacttg attctgcgag attccgtttc atgctgagcg aatactttgg cgcagcgcct 4140

caaaacgtac acgcgcatat tatcggagag cacggcgaca cagagcttcc tgtttggagc 4200

cacgcgaatg tcggcggtgt gccggtcagt gaactcgttg agaaaaacga tgcg 4254

<210> 5
<211> 10340
<212> DNA
<213> Artificial Sequence

<220>
<223> expression plasmid

<400> 5
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcc ttcaatcctt ttaataacaa ttatagcatc taatcttcaa caaactggcc 240

cgtttgttga actactcttt aataaaataa tttttccgtt cccaattcca cattgcaata 300

atagaaaatc catcttcatc ggctttttcg tcatcatctg tatgaatcaa atcgccttct 360

tctgtgtcat caaggtttaa ttttttatgt atttctttta acaaaccacc ataggagatt 420

aaccttttac ggtgtaaacc ttcctccaaa tcagacaaac gtttcaaatt cttttcttca 480

tcatcggtca taaaatccgt atcctttaca ggatattttg cagtttcgtc aattgccgat 540

tgtatatccg atttatattt atttttcggt cgaatcattt gaacttttac atttggatca 600

tagtctaatt tcattgcctt tttccaaaat tgaatccatt gtttttgatt cacgtagttt 660

tctgtattct taaaataagt tggttccaca cataccaata catgcatgtg ctgattataa 720

gaattatctt tattatttat tgtcacttcc gttgcacgca taaaaccaac aagattttta 780

ttaatttttt tatattgcat cattcggcga aatccttgag ccatatctga caaactctta 840

tttaattctt cgccatcata aacattttta actgttaatg tgagaaacaa ccaacgaact 900

gttggctttt gtttaataac ttcagcaaca accttttgtg actgaatgcc atgtttcatt 960

gctctcctcc agttgcacat tggacaaagc ctggatttac aaaaccacac tcgatacaac 1020

tttctttcgc ctgtttcacg attttgttta tactctaata tttcagcaca atcttttact 1080

ctttcagcct ttttaaattc aagaatatgc agaagttcaa agtaatcaac attagcgatt 1140

ttcttttctc tccatggtct cacttttcca ctttttgtct tgtccactaa aacccttgat 1200

ttttcatctg aataaatgct actattagga cacataatat taaaagaaac ccccatctat 1260

ttagttattt gtttagtcac ttataacttt aacagatggg gtttttctgt gcaaccaatt 1320

ttaagggttt tcaatacttt aaaacacata cataccaaca cttcaacgca cctttcagca 1380

actaaaataa aaatgacgtt atttctatat gtatcaagat aagaaagaac aagttcaaaa 1440

ccatcaaaaa aagacacctt ttcaggtgct ttttttattt tataaactca ttccctgatc 1500

tcgacttcgt tcttttttta cctctcggtt atgagttagt tcaaattcgt tctttttagg 1560

ttctaaatcg tgtttttctt ggaattgtgc tgttttatcc tttaccttgt ctacaaaccc 1620

cttaaaaacg tttttaaagg cttttaagcc gtctgtacgt tccttaagat tttacatttt 1680

tagaaatggg cgtgaaaaaa agcgcgcgat tatgtaaaat ataaagatta actaataagg 1740

aggacaaaca tgcaaaaact gctttctctg ccgtcaaatc ttgtccagtc ttttcatgaa 1800

cttgaaagag ttaaccgcac agattggttt tgcacatcag atccggttgg caaaaaactg 1860

ggctcaggcg gaggcacaag ctggttactg gaagaatgtt acaacgaata cagcgatgga 1920

gcgacatttg gcgaatggct ggaaaaagaa aaacgcattc tgttacatgc tggaggccaa 1980

agcagacgcc ttccgggata tgccccgtct ggcaaaatct taacaccggt cccggttttt 2040

agatgggaaa gaggacaaca tttaggccag aacctgctta gcttacaact gccgctttat 2100

gaaaaaatta tgtctctggc tccggataaa ctgcatacac tgattgcctc tggcgatgtg 2160

tatatccgca gcgaaaaacc gcttcagtct atcccggaag ctgatgttgt gtgctatgga 2220

ttatgggtcg atccgtctct ggcaacacat catggcgttt ttgcgtcaga tagaaaacat 2280

ccggaacaac tggattttat gctgcagaaa ccgtcacttg cagaattaga aagcctgtct 2340

aaaacacatt tatttctgat ggatattggc atctggttac tgtcagatag agcggtcgaa 2400

attctgatga aacgcagcca taaagaatca agcgaagaac ttaaatacta cgatctgtac 2460

tcagattttg gacttgcttt aggcacacat ccgcgcattg aagatgaaga agtgaataca 2520

cttagcgtcg caatcctgcc tcttccggga ggcgaatttt accattacgg aacatcaaaa 2580

gaactgattt cttcaacact tagcgttcaa aacaaagtgt atgatcagag acgcatcatg 2640

catagaaaag tgaaaccgaa tccggcaatg tttgtccaaa acgcggtcgt tcgcattccg 2700

ctttgcgctg aaaatgccga tttatggatt gaaaacagcc atatcggacc gaaatggaaa 2760

atcgcatcaa gacatattat cacaggcgtg ccggaaaatg attggtcact tgcagtcccg 2820

gcgggagtgt gtgtcgatgt ggtcccgatg ggagataaag gctttgttgc gagaccgtat 2880

ggattagatg atgtgtttaa aggcgatctg cgcgatagca aaacaacact tacaggcatt 2940

ccgtttggcg aatggatgtc taaaagagga ttatcataca cagatttaaa aggccgcaca 3000

gatgatctgc aggctgtttc agtgtttccg atggtgaaca gcgtcgaaga attaggcctg 3060

gtccttagat ggatgctttc tgaaccggaa ttagaagaag gaaaaaatat ttggctgcgc 3120

tctgaacatt tttcagcaga tgaaatctca gctggcgcca accttaaaag actgtacgcg 3180

caaagagaag aatttcgcaa aggaaattgg aaagcacttg cggttaacca tgaaaaatca 3240

gtgttttatc agctggatct ggcagatgca gcggaagatt ttgttcgcct tggcttagat 3300

atgccggaac ttttaccgga agatgctctg caaatgtcac gcattcataa tcgcatgctt 3360

agagcccgca tcctgaaact tgatggaaaa gattatagac cggaagaaca ggctgccttt 3420

gatctgctta gagatggatt actggatggc attagcaacc gcaaatctac accgaaactg 3480

gatgtctatt ctgatcaaat tgtttggggc agatcaccgg tgcgcatcga tatggcaggc 3540

ggctggacag atacaccgcc gtatagcctt tattctggag gcaatgttgt gaaccttgcg 3600

attgaattaa atggccaacc gccgcttcag gtctacgtta aaccgtgcaa agattttcat 3660

atcgttctga gatcaatcga tatgggagct atggaaatcg tgagcacatt tgatgaactg 3720

caagattaca agaaaattgg ctcaccgttt agcatcccga aagcagcgtt aagcctggcc 3780

ggatttgctc cggccttttc tgctgtcagc tatgcatcac tggaagaaca gcttaaagat 3840

tttggcgcag gaattgaagt tacactttta gctgccatcc cggcgggctc tggattaggc 3900

acaagctcta ttctggcttc aacagtgctt ggagccatca atgatttttg cggcctggca 3960

tgggataaaa acgaaatttg tcaaagaaca cttgtcttag aacagctgct tacaacaggc 4020

ggcggctggc aagatcagta tggcggagtc ctgcaaggag ttaaactgct gcagacagaa 4080

gctggctttg cccagtcacc gttagttcgc tggctgccgg atcatctttt tacacatccg 4140

gaatacaaag attgtcatct tttatactac acaggaatta caagaacagc aaaaggcatt 4200

ttagcggaaa tcgtgtcaag catgtttctg aatagctcac tgcatcttaa cctgcttagc 4260

gaaatgaaag cacatgcgct tgatatgaat gaagctattc aaagaggctc ttttgtcgaa 4320

tttggacgct tagttggcaa aacatgggaa cagaataaag ccctggatag cggaacaaac 4380

ccgccggcag tggaagcgat catcgatctg atcaaagatt acacactggg atacaaactg 4440

cctggagcag gcggcggcgg ctatctgtat atggttgcga aagatccgca agcagctgtg 4500

agaattagaa aaatcctgac agaaaatgct ccgaacccga gagcccgctt tgtggaaatg 4560

acactttcag ataaaggatt tcaggtctca agaagctaag ctcttaagga ggattttaga 4620

atgggttcta tcatcagact tcagggtggt ttaggcaatc aactgtttca atttagcttt 4680

ggttatgctc tttcaaaaat caatggcaca ccgctgtact ttgatatttc acattacgca 4740

gaaaacgatg atcatggcgg atatagactg aataaccttc aaatcccgga agaatatctt 4800

cagtactaca caccgaaaat caacaacatc tacaaactgc ttgtcagagg cagccgctta 4860

tatccggata tttttctgtt tcttggcttt tgcaacgaat ttcatgcgta tggctatgat 4920

tttgaataca tcgcgcaaaa atggaaaagc aaaaaataca tcggatactg gcagtctgaa 4980

catttctttc ataaacatat tctggatctg aaagaatttt tcatcccgaa aaacgttagc 5040

gaacaagcta acttactggc agcgaaaatc ttagaatctc agtcaagcct gtcaattcat 5100

atcagacgcg gcgattacat caaaaacaaa acagccacac tgacacatgg agtttgctct 5160

cttgaatact acaagaaagc actgaacaaa atcagagatt tagccatgat tcgcgatgtg 5220

tttatctttt ctgatgatat tttctggtgt aaagaaaaca tcgaaacact tctgagcaaa 5280

aaatacaaca tctactactc agaagattta agccaagaag aagatttatg gctgatgtct 5340

ctggctaatc atcatattat cgccaactct tcattttcat ggtggggcgc atatcttgga 5400

agctctgcga gccagattgt tatctatccg acaccgtggt atgatattac accgaaaaac 5460

acatacattc ctatcgtgaa ccattggatc aacgtggaca aacattcctc ctgctaaaaa 5520

ggaggaacta ctatgggcaa tacatcaatt caaacacaga gctatagagc agttgataaa 5580

gatgcgggac aaagccgctc ttatattatc ccgtttgcac tgctttgctc tctgtttttc 5640

ctgtgggctg tggccaataa cctgaatgat attttactgc cgcagtttca acaggcgttt 5700

acacttacaa actttcaagc tggattaatt cagagcgcct tttactttgg ctactttatc 5760

atcccgatcc cggctggaat ccttatgaaa aaactgtctt acaaagctgg aattatcaca 5820

ggcttatttc tgtatgcact gggagcagct ctgttttggc ctgctgccga aatcatgaat 5880

tatacactgt ttctggtcgg actgtttatt atcgcagcgg gactgggctg tcttgaaaca 5940

gctgccaacc cgtttgtcac agttcttgga ccggaatcaa gcggccattt tagactgaac 6000

cttgcacaaa catttaactc atttggagca atcatcgcgg ttgtgtttgg ccagagctta 6060

attctgtcta atgtcccgca tcaaagccag gatgttctgg ataaaatgtc tccggaacaa 6120

ctttcagcgt ataaacattc acttgtgtta agcgtccaga caccgtatat gattatcgtt 6180

gcaatcgtgc ttttagtcgc gctgcttatt atgttaacaa aatttccggc actgcaatct 6240

gataaccatt cagatgcgaa acagggctca tttagcgctt ctttatcaag actggccaga 6300

attagacatt ggcgctgggc tgtgttagcc caattttgct atgtcggagc acagacagcg 6360

tgttggtcat atctgatccg ctatgctgtc gaagaaattc cgggaatgac agccggcttt 6420

gcagcgaatt atcttacagg cacaatggtt tgctttttca tcggaagatt tacaggcaca 6480

tggttaattt ctcgctttgc accgcataaa gtgcttgctg cctatgcgtt aatcgcaatg 6540

gcgctgtgcc ttatttcagc ttttgccggc ggacatgtcg gccttattgc attaacactg 6600

tgtagcgcgt ttatgtctat ccaatacccg acaatcttta gcctgggaat taaaaacctt 6660

ggccaggata caaaatacgg atcttcattt atcgttatga caattatcgg cggaggcatt 6720

gttacaccgg tgatgggatt tgtgagcgat gcagcgggca atatcccgac agctgaatta 6780

attccggctc tgtgttttgc cgttatcttt atctttgcta gatttcgctc tcaaacagcc 6840

acaaactaat ctagagtcga cgtccaggca tcaaataaaa cgaaaggctc agtcgaaaga 6900

ctgggccttt cgttttatct gttgtttgtc ggtgaacgct ctctactaga gtcacactgg 6960

ctcaccttcg ggtgggcctt tctgcgttta tatacgcgtt aacccgggcc cgcggatgga 7020

tatgatcaga tcctttaact ctggcaaccc tcaaaattga atgagacatg ctacacctcc 7080

ggataataaa tatatataaa cgtatataga tttcataaag tctaacacac tagacttatt 7140

tacttcgtaa ttaagtcgtt aaaccgtgtg ctctacgacc aaaactataa aacctttaag 7200

aactttcttt ttttacaaga aaaaagaaat tagataaatc tctcatatct tttattcaat 7260

aatcgcatcc gattgcagta taaatttaac gatcactcat catgttcata tttatcagag 7320

ctcgtgctat aattatacta attttataag gaggaaaaaa tatgggcatt tttagtattt 7380

ttgtaatcag cacagttcat tatcaaccaa acaaaaaata agtggttata atgaatcgtt 7440

aataagcaaa attcatataa ccaaattaaa gagggttata atgaacgaga aaaatataaa 7500

acacagtcaa aactttatta cttcaaaaca taatatagat aaaataatga caaatataag 7560

attaaatgaa catgataata tctttgaaat cggctcagga aaaggccatt ttacccttga 7620

attagtaaag aggtgtaatt tcgtaactgc cattgaaata gaccataaat tatgcaaaac 7680

tacagaaaat aaacttgttg atcacgataa tttccaagtt ttaaacaagg atatattgca 7740

gtttaaattt cctaaaaacc aatcctataa aatatatggt aatatacctt ataacataag 7800

tacggatata atacgcaaaa ttgtttttga tagtatagct aatgagattt atttaatcgt 7860

ggaatacggg tttgctaaaa gattattaaa tacaaaacgc tcattggcat tacttttaat 7920

ggcagaagtt gatatttcta tattaagtat ggttccaaga gaatattttc atcctaaacc 7980

taaagtgaat agctcactta tcagattaag tagaaaaaaa tcaagaatat cacacaaaga 8040

taaacaaaag tataattatt tcgttatgaa atgggttaac aaagaataca agaaaatatt 8100

tacaaaaaat caatttaaca attccttaaa acatgcagga attgacgatt taaacaatat 8160

tagctttgaa caattcttat ctcttttcaa tagctataaa ttatttaata agtaagttaa 8220

gggatgcata aactgcatcc cttaacttgt ttttcgtgtg cctatttttt gtgaatcgat 8280

tcagctgcat taatgaatcg gccaacgcgc ggggagaggc ggtttgcgta ttgggcgctc 8340

ttccgcttcc tcgctcactg actcgctgcg ctcggtcgtt cggctgcggc gagcggtatc 8400

agctcactca aaggcggtaa tacggttatc cacagaatca ggggataacg caggaaagaa 8460

catgtgagca aaaggccagc aaaaggccag gaaccgtaaa aaggccgcgt tgctggcgtt 8520

tttccatagg ctccgccccc ctgacgagca tcacaaaaat cgacgctcaa gtcagaggtg 8580

gcgaaacccg acaggactat aaagatacca ggcgtttccc cctggaagct ccctcgtgcg 8640

ctctcctgtt ccgaccctgc cgcttaccgg atacctgtcc gcctttctcc cttcgggaag 8700

cgtggcgctt tctcatagct cacgctgtag gtatctcagt tcggtgtagg tcgttcgctc 8760

caagctgggc tgtgtgcacg aaccccccgt tcagcccgac cgctgcgcct tatccggtaa 8820

ctatcgtctt gagtccaacc cggtaagaca cgacttatcg ccactggcag cagccactgg 8880

taacaggatt agcagagcga ggtatgtagg cggtgctaca gagttcttga agtggtggcc 8940

taactacggc tacactagaa gaacagtatt tggtatctgc gctctgctga agccagttac 9000

cttcggaaaa agagttggta gctcttgatc cggcaaacaa accaccgctg gtagcggtgg 9060

tttttttgtt tgcaagcagc agattacgcg cagaaaaaaa ggatctcaag aagatccttt 9120

gatcttttct acggggtctg acgctcagtg gaacgaaaac tcacgttaag ggattttggt 9180

catgagatta tcaaaaagga tcttcaccta gatcctttta aattaaaaat gaagttttaa 9240

atcaatctaa agtatatatg agtaaacttg gtctgacagt taccaatgct taatcagtga 9300

ggcacctatc tcagcgatct gtctatttcg ttcatccata gttgcctgac tccccgtcgt 9360

gtagataact acgatacggg agggcttacc atctggcccc agtgctgcaa tgataccgcg 9420

agacccacgc tcaccggctc cagatttatc agcaataaac cagccagccg gaagggccga 9480

gcgcagaagt ggtcctgcaa ctttatccgc ctccatccag tctattaatt gttgccggga 9540

agctagagta agtagttcgc cagttaatag tttgcgcaac gttgttgcca ttgctacagg 9600

catcgtggtg tcacgctcgt cgtttggtat ggcttcattc agctccggtt cccaacgatc 9660

aaggcgagtt acatgatccc ccatgttgtg caaaaaagcg gttagctcct tcggtcctcc 9720

gatcgttgtc agaagtaagt tggccgcagt gttatcactc atggttatgg cagcactgca 9780

taattctctt actgtcatgc catccgtaag atgcttttct gtgactggtg agtactcaac 9840

caagtcattc tgagaatagt gtatgcggcg accgagttgc tcttgcccgg cgtcaatacg 9900

ggataatacc gcgccacata gcagaacttt aaaagtgctc atcattggaa aacgttcttc 9960

ggggcgaaaa ctctcaagga tcttaccgct gttgagatcc agttcgatgt aacccactcg 10020

tgcacccaac tgatcttcag catcttttac tttcaccagc gtttctgggt gagcaaaaac 10080

aggaaggcaa aatgccgcaa aaaagggaat aagggcgaca cggaaatgtt gaatactcat 10140

actcttcctt tttcaatatt attgaagcat ttatcagggt tattgtctca tgagcggata 10200

catatttgaa tgtatttaga aaaataaaca aataggggtt ccgcgcacat ttccccgaaa 10260

agtgccacct gacgtctaag aaaccattat tatcatgaca ttaacctata aaaataggcg 10320

tatcacgagg ccctttcgtc 10340

## Description

The present invention relates to the technical fields of genetic engineering, particularly to genetic engineering of *Bacillus* cells for the production of fucosylated oligosaccharides, and to the microbial production of fucosylated oligosaccharides using said genetically engineered *Bacillus* cells.

### Background

Human breast milk has optimal nutritional properties for infants. The saccharides that are present in human breast milk represent the main component of human milk, ahead of fats and proteins. In addition to lactose, which serves as an energy source, human breast milk contains about 5 to 25 grams per liter of complex sugar molecules, i.e. oligosaccharides. These oligosaccharides are found at significant concentrations in only human milk, and they are collectively known as human milk oligosaccharides (HMOs).

Approximately 200 structurally distinct HMOs have been identified to date. Said HMOs are based on the disaccharide lactose (consisting of a glucose (Glc) moiety and a galactose (Gal) moiety) and bear additional monosaccharide residues which are based on N-acetyl-glucosamine (GlcNAc), fucose (Fuc), sialic acid (NeuNAc), and galactose (Gal). The concentration and composition of HMOs in human milk varies between individuals and during the lactation period from up to 20 g/L in the colostrum to 5-10 g/L in the mature milk.

Milk of women belonging to the so-called "secretor phenotype" contains a high content of α-1,2-fucosylated HMOs. These women express the *FUT2* gene encoding the so-called "fucosyltransferase 2". The most abundant HMOs in their milk are 2'-fucosyllactose (2'-FL; Fuc(α1-2)Gal(β1-4)Glc) and lacto-*N*-fucopentaose-I (LNPF-I; Fuc(α1-2)Gal(β1-3)GlcNAc(1-3)Gal(β1-4)Glc).

Human milk oligosaccharides are not digested during their transit through the intestine of infants. Due to their persistence in the infant's gut, they exhibit beneficial effects. More specifically, HMOs have been shown to be prebiotic as they serve as carbon source for commensal microorganisms of the genera *Bifidobacterium, Bacteroides* and *Lactobacillus.* Therefore, HMOs support proliferation of these microorganisms in infants' guts.

Human milk oligosaccharides also directly reduce colonization of an infant's gut by pathogens in that they prevent adherence of said pathogens to glycan structures on the gut's mucosal surface. The HMOs function as a decoy due to their structural similarity to epithelial surface glycans and inhibit invasion of the pathogens thereby reducing the risk of infections.

Alpha-1,2-fucosylated HMOs have been shown to be protective against infections with *Campylobacter jejuni,* the causative agent of most common bacterial diarrheas. The α-1,2-fucosylated HMOs are also associated with protection against diarrhea caused by the heat stable toxin of *Escherichia coli.* Also, the risk of infections with diarrhea-mediating caliciviruses is reduced by a high content of α-1,2-fucosylated HMOs in breast milk. HMOs, especially the fucosylated HMO lacto-*N*-fucopentaose V (LNFP-V; Gal(β1-3)GlcNAc(β1-3)Gal(β1-4)[Fucα1-3]Glc), bind(s) to the carbohydrate binding site of toxin A from *Clostridium difficile,* the most common cause of nosocomial diarrhea. Thus, HMOs seem to prevent the interaction of toxin A from *C*. *difficile* with cellular receptors.

Furthermore, adherence of *Pseudomonas aeruginosa* to epithelial cells was significantly inhibited by 2'-FL and 3-fucosyllactose (3-FL; Gal(β1-4)[Fucα1-3]Glc). Binding of noroviruses (Norwalk-like viruses, NLV), the main cause of acute gastroenteritis, to histo-blood group antigens is prevented by α-1,2-fucosylated HMOs as well as by α-1,3-fucosylated HMOs. This indicates the potential of these HMOs to inhibit norovirus capsid-binding to host receptor glycans.

Due to the known benefits of HMOs, and especially of fucosylated HMOs, an economically worthwhile process for their synthesis is desired such that these oligosaccharides or at least some of these oligosaccharides become available as supplement for infant formula.

Due to the limited supply and difficulties of obtaining pure fractions of individual human milk oligosaccharides, chemical routes to the synthesis of some of these complex molecules were developed. However, neither chemical synthesis nor biocatalytic approaches proved to be commercially sustainable. Moreover, particularly chemical synthesis of human milk oligosaccharides involves the use of several noxious chemicals, which impose the risk to contaminate the final product.

Due to the challenges involved in the chemical synthesis of human milk oligosaccharides, fermentative approaches for producing HMOs were developed. Today, microbial production of several HMOs such as 2'-fucosyllactose, 3-fucosyllactose, lacto-*N*-tetraose, lacto-*N*-neotetraose, lacto-*N*-fucopentaose I, lacto-*N*-difucohexaose II, 3'-sialyllactose and 6'-sialyllactose using mainly genetically engineered *Escherichia coli* strains have been disclosed.

To date, recombinant *E. coli* cells are used for microbial production of HMOs in industrial scale. However, the genus *Escherichia coli* comprises pathogenic members as well as non-pathogenic members. Notwithstanding that non-pathogenic *E. coli* strains are employed for the microbial production of HMOs, such non-pathogenic *E. coli* are not recognized as safe for the manufacturing of products that are intended for human consumption in a variety of jurisdictions. Therefore, microbial cells of genera that are recognized as safe in such jurisdictions are needed for the manufacturing of compounds that are intended for human consumption to obtain regulatory approval of said products for their commercialization in said jurisdictions.

The problem is solved by using bacterial cells of the genus *Bacillus* which are recognized as being safe for human consumption, and which are capable of producing a fucosylated oligosaccharide.

Bacteria of the genus *Bacillus* are gram-positive, rod-shaped, endospore-forming microbial cells of either aerobic or facultatively anaerobic species. The genus *Bacillus* belongs to the phylum Firmicutes. The genome of the members of the genus *Bacillus* possesses a bias towards A-T base pairs in its codon usage. *Bacillus* species are almost ubiquitously present in nature. They can for example be found in soil (*B. subtilis*), but also occur in extreme environments such as high pH (*B. alcalophilus*), high temperature (*B. thermophilus*), or high salt (*B*. *halodurans*).

The genus *Bacillus* includes 266 named species which include free living species as well as parasitic pathogenic species. Two *Bacillus* species are considered of being medically significant: *B*. *anthracis,* which causes anthrax, and *B. cereus,* which causes food poisoning. A third species, *B*. *thuringiensis,* is an important insect pathogen producing a toxin that can kill insects. Thus, it is used as an insecticide to control insect pests.

Because of their GRAS (Generally Recognized as Safe) status, several *Bacillus* species, e.g. *B. amyloliquefaciens, B. licheniformis and B. subtilis,* are used in the biotechnological production of various proteins and compounds to be used in food and pharmaceutical industry.

*B. amyloliquefaciens* is the source of restriction enzyme BamHI, and also synthesizes the natural antibiotic protein barnase. In addition, *B*. *amyloliquefaciens* produces plantazolicin, an antibiotic with selective activity against *B.anthracis.* Alpha-amylase from *B*. *amyloliquefaciens* is often used in starch hydrolysis. *B*. *amyloliquefaciens* is also a source of subtilisin, which catalyzes the breakdown of proteins.

*B. amyloliquefaciens* is a root-colonizing bacterium that is used to fight some plant root pathogens in agriculture, aquaculture and hydroponics as it acts against bacterial and fungal pathogens, and may prevent infection by competitive exclusion or out-competing the unwanted pathogen.

Its high capacity of secreting alkaline serine protease has made *B*. *licheniformis* one of the most important bacteria in industrial enzyme production. Subtilisin Carlsberg secreted by *B. licheniformis* is used as a detergent protease, and is sold under the trade name Alcalase®.

*B. subtilis* is a catalase positive bacterium that is found in soil and the gastrointestinal tract of ruminants and humans. *B. subtilis* and substances derived from this endotoxin-free bacterium have been evaluated by different authoritative bodies for their safe and beneficial use in food. In the United States carbohydrase and protease enzymes from *B. subtilis* are recognized as generally safe (GRAS) by the US Food and Safety Administration (FDA). *Bacillus subtilis* has also been granted the "Qualified Presumption of Safety" status by the European Food Safety Authority.

Moreover, nontoxigenic and nonpathogenic strains of *B. subtilis* strain were commonly used in food. For example, fermented soy beans in the form of Natto, which is commonly consumed in Japan, contains as many as 10⁸ viable *B. subtilis* cells per gram. The fermented beans are recognized for their contribution to a healthy gut flora and vitamin K₂ intake. The natto product and the *B. subtilis var. natto* as its principal component are FOSHU (Foods for Specified Health Use) approved by the Japanese Ministry of Health, Labor and Welfare as effective for preservation of health.

*B. subtilis* is easy to handle, grows rapidly and the culture conditions are simple. Recombinant *B. subtilis* strains are used in the production of polyhydroxyalkano-lates, hyaluronic acid and various enzymes such as amylase and proteases.

Wild-type natural isolates of *B. subtilis* are difficult to work with as compared to laboratory strains that have undergone domestication processes of mutagenesis and selection. These domesticated strains often have improved capabilities to undergo natural competence development (uptake and integration of environmental DNA), growth, and loss of abilities needed "in the wild". In *B. subtilis,* linear DNA as well as multimeric forms of plasmid DNA are actively taken up by natural competent cells.

Under certain physiological conditions, a small subpopulation of *B. subtilis* cells becomes competent. In *B. subtilis,* natural competence is regulated by a complex regulatory network. Key regulators in this network are, amongst others, the master regulator of competence ComK and the transcriptional master regulator of sporulation SpoOA. Transformation efficiency of *B. subtilis* cells and probably the efficacy for integrating DNA into their genome can be improved by genetic engineering. This can be achieved by ectopic integration of an expression cassette, comprising a regulated promoter (e.g. the mannitol-inducible P*_{mtlA}* promoter) and the genes *comK* and *comS,* into the genome of *B. subtilis.* Additionally, this strategy allows transformation of *B. subtilis* by natural competence using a complex medium (e.g. LB).

For the production of fucosylated saccharides, *B. subtilis* can be genetically modified by various methods.

Gene integration and/or (simultaneous) gene inactivation by disruption or deletion can be achieved by homologous recombination. For an efficient homologous recombination, at least 400-500 bp of homology arms are necessary in *B. subtilis.*

Another method for targeted genome engineering is the modern CRISPR-Cas9 system. This rapid and markerless genome-editing tool can be used for large scale genomic deletions, small and large DNA insertions, gene silencing by introduction of a stop codon as well as introduction of point mutations. No previous genome modifications are required for the scarless genome editing by CRISPR-Cas9.

Random chromosomal integration of genes and insertional mutagenesis can be performed using a modified mariner-derived transposon. This system is not biased toward hotpots in *B. subtilis* while showing high efficiency in random ectopic integration.

Although *Bacillus* species are used for industrial scale production of enzymes, *Bacillus* cells have not been implemented to date for industrial scale production of oligosaccharides, in particular of fucosylated oligosaccharides.

Chinese patent application CN 108 410 787 A discloses recombinant *Bacillus subtilis* cells which synthesizes lactyl-N-neotetraose. Said recombinant *B. subtilis* cells possess a lactose permease gene which has been integrated into the cell's genome. In addition, said *Bacillus* cell which bears a plasmid comprising a beta-1,3-*N*-glucosamine transferase gene and a β-1,4-galactosyltransferase gene. The *B. subtilis* cells may be cultivated in the presence of exogenous lactose, and synthesizes lactyl-N-neotetraose at titers of up to about 1 g/L which is too little for economically reasonable industrial scale production.

Although a variety of patent applications mention *Bacillus* as a genus which is suitable for the production of oligosaccharides such as lacto-*N*-neotetraose, no commercial use of *Bacillus* for the productions of fucosylated oligosaccharides, in particular of fucosylated human milk oligosaccharides has been implemented yet, presumably because of the significant efforts in metabolic engineering that are required to implement the necessary biosynthetic pathways for HMO production in *Bacillus.* Whereas above-referenced *B. subtilis* for the production of LNnT relies on donor substrates that are naturally occurring in *B. subtilis* cells, production of a fucosylated oligosaccharide in *Bacillus* requires implementation of a heterologous metabolic pathway in the cell for providing the required donor substrate GDP-fucose.

Chinese patent application CN 109 735 479 A discloses recombinant *Bacillus subtilis* cells for synthesizing 2'-fucosyllactose, wherein the expression of a lactose transport enzyme is enhanced, and which cell expresses a fucose kinase, a phosphate guanine transferase and a fucosyltransferase. The yield of 2'-fucosyllactose in the fermentation medium was reported to be between 0.424 g/L and 1.042 g/L.

It was nonetheless an object of the present invention to provide a bacterial cell of the genus *Bacillus* for the production of fucosylated oligosaccharides.

The object has been achieved by providing a *Bacillus* cell that possesses a lactose permease for import of exogenous lactose into the cell, a *de novo* GDP-fucose biosynthesis pathway for providing GDP-fucose, and a fucosyltransferase for transfer of a fucose moiety from GDP-fucose to lactose. Said *Bacillus* cell can be cultivated in the presence of exogenous lactose to produce a fucosylated oligosaccharide.

### Summary

According to a first aspect, provided is a non-sporulating bacterial cell of the genus *Bacillus* for the production of a fucosylated oligosaccharide, wherein the *Bacillus* cell possesses a lactose permease, a GDP-fucose biosynthesis pathway, and a fucosyltransferase.

According to a second aspect, provided is the use of the non-sporulating bacterial cell of the genus *Bacillus* according to the first aspect for the production of a fucosylated oligosaccharide.

According to a third aspect, provided is a method for the production of a fucosylated oligosaccharide, the method comprising:
- providing a non-sporulating bacterial cell of the genus *Bacillus,* wherein said *Bacillus* cell possesses a lactose permease, a GDP-fucose biosynthetic pathway, and a lactose-accepting fucosyltransferase;
- cultivating the *Bacillus* cell in a culture medium containing lactose and under conditions that are permissive for the *Bacillus* cell to produce the fucosylated oligosaccharide, and
- optionally retrieving the fucosylated oligosaccharide from the culture medium and/or the *Bacillus* cell.

According to a fourth aspect, provided are fucosylated oligosaccharides that were produced by a *Bacillus* cell which possesses a lactose permease, a GDP-fucose biosynthesis pathway and a fucosyltransferase.

According to a fifth aspect, provided is the use of a fucosylated oligosaccharide that was produced by a *Bacillus* cell which possesses a lactose permease, a GDP-fucose biosynthesis pathway and a fucosyltransferase for the manufacturing of a nutritional composition.

According to a sixth aspect, provided are nutritional compositions containing at least one fucosylated oligosaccharide that had been produced by a *Bacillus* cell which possesses a lactose permease, a GDP-fucose biosynthesis pathway and a fucosyltransferase.

### Detailed description

The present invention concerns *Bacillus* cells for the production of fucosylated oligosaccharides, their use and methods for producing fucosylated oligosaccharides by cultivating said *Bacillus* cells in a culture medium containing lactose, and under conditions that are permissive for the production of said fucosylated oligosaccharide by said *Bacillus* cell.

For being able to produce a fucosylated oligosaccharide, a *Bacillus* cell has to provide a donor substrate comprising a fucose moiety, and an acceptor substrate being a disaccharide or an oligosaccharide to a fucosyltransferase such that the fucosyltransferase can transfer the fucose moiety from the donor substrate to said acceptor substrate thereby generating the fucosylated oligosaccharide.

It is to be understood that the fucosylated oligosaccharide, said *Bacillus* cell is intended to produce, is the desired fucosylated oligosaccharide, whereas other fucosylated oligosaccharides that may be generated due to the promiscuity of the fucosyltransferase during production of the desired fucosylated oligosaccharide are considered as undesired fucosylated oligosaccharides or by-products. A suitable donor substrate is GDP-L-fucose, and a suitable acceptor substrate for generating a fucosyllactose is the disaccharide lactose. The resulting desired fucosylated oligosaccharide is 2'-fucosyllactose, 3-fucosyllactose or 2',3-difucosyllactose.

Wild-type *Bacillus* cells neither synthesize lactose intracellularly nor do they internalize and metabolize exogenous lactose. However, lactose is the acceptor substrate for a fucose moiety by a lactose-accepting fucosyltransferase in the formation of some fucosylated oligosaccharides. Hence, to be able to produce a fucosylated oligosaccharide such as 2'-fucosyllactose, 3-fucosyllactose or 2',3-difucosyllactose, a *Bacillus* cell has to have the capability of providing lactose to the lactose-accepting fucosyltransferase, either by generating lactose intracellularly and/or by internalizing exogenous lactose.

In an embodiment, the *Bacillus* cell for producing a fucosylated oligosaccharide is able to internalize exogenous lactose by possessing a lactose permease. The term "exogenous" with respect to lactose as used herein refers to lactose that does not originate from the *Bacillus* cell, i.e. being intracellularly synthesized by the *Bacillus* cell, but which originates from outside the *Bacillus* cell and is added to the culture medium in which the *Bacillus* cell is grown to produce the fucosylated oligosaccharide.

In an additional and/or alternative embodiment, the *Bacillus* cell has been genetically engineered to be able to internalize exogenous lactose, i.e. to possess a lactose permease. Thus, the *Bacillus* cell for producing a fucosylated oligosaccharide possesses a heterologous lactose permease. A suitable lactose permease is *E. coli* LacY or a functional variant thereof.

The term "heterologous" as used herein with respect to proteins, polypeptides, enzymes and transporters, but also with respect to nucleic acid molecules and/or nucleotide sequences, refers to a molecule that is non-native to the species of cell which contains said molecule. The term "non-native" indicates that said molecule is not present in the naturally occurring or wild-type progenitor cell, i.e. the cell of the same species that is most commonly occurring in nature, of the *Bacillus* cell.

The term "functional variant" as used herein with respect to enzymes and/or transport molecules refers to proteins or polypeptides possessing the same activity (enzymatic, catalytic or translocating) as the referenced enzyme or transporter, but which has a different amino acid sequence than the referenced enzyme or transporter molecule. Thus, a typical variant of a protein/polypeptide differs in amino acid sequence from the reference protein/polypeptide. A variant and the reference protein/polypeptide may differ in amino acid sequence by one or more substitutions, additions, and/or deletions in any combination. Hence, the term "functional variant" comprises truncated versions of the referenced protein/polypeptide which possesses the same activity as the reference protein/polypeptide. A substituted or inserted amino acid residue may or may not be one encoded by the genetic code. A variant of a protein/ polypeptide may be a naturally occurring such as an allelic variant, or it may be a variant that is not known to occur naturally. Non-naturally occurring variants of proteins/polypeptides may be made by mutagenesis techniques, by direct synthesis, and by other recombinant methods known to the persons skilled in the art. Within the scope of the present disclosure, also proteins and interspecies homologs are comprised by the term "variant", that have an amino acid sequence/ nucleic acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino acid sequence identity, preferably over a region of at least about 25, 50, 100, 200, 500, 1000, or more amino acids, to the referenced polypeptide.

The term "same activity" as used herein refers to an enzymatic, catalytic or transporting activity of a protein/polypeptide in qualitative manner only. Hence, "functional variant" also comprises variants which possess an increased or decreased activity as compared to the activity of the referenced protein/polypeptide.

In an additional and/or alternative embodiment, the *Bacillus* cell has been genetically engineered to contain and express a nucleotide sequence which encodes a lactose permease, preferably a nucleotide sequence that encodes the *E. coli* lactose permease LacY or a functional variant thereof.

The *E. coli* lactose permease LacY is encoded by the protein coding region (i.e. the open reading frame) of the *E*. *coli lac*Y gene (Gen Bank accession number: NP_414877.1). Hence, in an embodiment, the *Bacillus* cell has been genetically engineered to contain and express a nucleotide sequence that encodes the *E. coli* lactose permease LacY or a functional variant thereof.

In an additional and/or alternative embodiment, the codon usage of the nucleotide sequence encoding the heterologous lactose permease is adapted to the codon usage of *Bacillus.* The codon usage of *B. subtilis* for example is peculiar in that the overall GC-content is below about 45 %, the GC-content of the first letter of the codons above about 51 %, the CG-content of the second letter of the codons below about 36.1 %, and the CG-content of the third letter of the codons below about 46 %.

For expression of the lactose permease, the *Bacillus* cell contains a recombinant lactose permease gene, wherein the nucleotide sequence which encodes the lactose permease is operably linked to expression control sequences which mediate expression of the lactose permease open reading frame.

The term "operably linked" as used herein, shall mean a functional linkage between a nucleic acid/gene expression control sequence (such as a promoter, signal sequence, or array of transcription factor binding sites) and a second nucleic acid sequence (typically referred to as "protein coding region", "open reading frame", and sometimes even as "gene"), wherein the expression control sequence effects transcription and/or translation of the nucleotide sequence corresponding to the second sequence. Accordingly, the term "promoter" designates DNA sequences which usually "precede" an open reading frame in a DNA polymer and provide a site for initiation of the transcription into mRNA. "Regulator" DNA sequences, also usually "upstream" of (i.e., preceding) an open reading frame in a given DNA polymer, bind proteins that determine the frequency (or rate) of transcriptional initiation. Collectively referred to as "promoter/regulator" or "control" DNA sequence, these sequences which precede a selected open reading frame (or series of open reading frames) in a functional DNA polymer cooperate to determine whether the transcription (and eventual expression) of an open reading frame will occur. DNA sequences which "follow" a gene in a DNA polymer and provide a signal for termination of the transcription into mRNA are referred to as transcription "terminator" sequences.

The recombinant lactose permease gene may be integrated into the *Bacillus* chromosome, or present as an episomal version on a plasmid within the *Bacillus* cell.

Expression of the heterologous lactose permease gene in the *Bacillus* cell enables the resulting *Bacillus* cell to internalize exogenously supplied lactose from the culture medium. The internalized lactose can then serve as an acceptor substrate for a fucosyltransferase (see herein below).

The *Bacillus* cell for producing a fucosylated oligosaccharide has to be able to provide a donor substrate for transferring a fucose moiety to an acceptor substrate. The donor substrate for a fucose moiety is GDP-fucose. Hence, the *Bacillus* cell has to be able to intracellularly produce GDP-fucose. For intracellular biosynthesis of GDP-fucose, the *Bacillus* cell possesses a GDP-fucose biosynthesis pathway. The *Bacillus* has been genetically engineered to possess a GDP-fucose biosynthesis pathway. The GDP-fucose biosynthesis pathway may either be a *de novo* GDP-fucose biosynthesis pathway and/or a GDP-fucose salvage pathway.

In an embodiment, the *Bacillus* cell possesses a *de novo* GDP-fucose biosynthesis pathway for intracellular biosynthesis of GDP-fucose. The *Bacillus* cell has been genetically engineered to possess the *de novo* GDP-fucose biosynthesis pathway.

The *de novo* GDP-fucose biosynthesis pathway comprises activities of the following enzymes:
1. a fructose-6-phosphate isomerase;
2. a phosphomannomutase;
3. a GDP:mannose-1-phosphate guanylyltransferase;
4. a GDP-mannose-4,6-dehydratase; and
5. a GDP-fucose synthase.

Hence, the *Bacillus* cell for producing a fucosylated oligosaccharide possesses a fructose-6-phosphate isomerase (typically also designated as mannose-6-phosphate isomerase), a phosphomannomutase, a GDP:mannose-1-phosphate guanylyltransferase, a GDP-mannose-4,6-dehydratase, and a GDP-fucose synthase.

In an additional and/or alternative embodiment, the *Bacillus* cell has been genetically engineered to possess a fructose-6-phosphate isomerase, a phosphomannomutase, a GDP:mannose-1-phosphate guanylyltransferase, a GDP-mannose-4,6-dehydratase, and a GDP-fucose synthase.

The *de novo* pathway of GDP-fucose biosynthesis starts from isomerization of fructose-6-phosphate to mannose-6-phosphate, a reaction that is catalyzed by the fructose-6-phosphate isomerase. In an additional and/or alternative embodiment, the *Bacillus* cell has been genetically engineered to contain and express a nucleotide sequence which encodes a fructose-6-phosphate isomerase. A suitable fructose-6-phosphate is *E. coli* ManA or a functional variant thereof. An exemplary nucleotide sequence is the nucleotide sequence which encodes *E. coli* ManA.

The *E. coli* fructose-6-phosphate isomerase ManA is encoded by the protein coding region of the *E*. *coli man*A gene (GenBank accession number: NP_416130.3). Thus, in an additional embodiment, the *Bacillus* cell has been genetically engineered to contain and express a nucleotide sequence which encodes the *E. coli* ManA or a functional variant thereof.

The *E. coli* fructose-6-phosphate isomerase ManA is encoded by the open reading frame of the *E*. *coli man*A gene (Gen Bank accession number: NP_416130.3). Thus, in an additional and/or alternative embodiment, the *Bacillus* cell contains the *E. coli man*A gene or a functional variant thereof.

In an additional and/or alternative embodiment, the codon usage of the nucleotide sequence encoding the fructose-6-phosphate isomerase is adapted to the codon usage of *Bacillus.*

Other suitable fructose-6-phosphate isomerases are *B. subtilis* ManA and the para-logous proteins *B. subtilis* GmuF (YdhS) and *B. subtilis* Pmi (Yvyl) or functional variants thereof. Exemplary nucleotide sequences are the nucleotide sequences which encodes *B. subtilis* ManA, *B. subtilis* GmuF or *B. subtilis* Pmi. The *B. subtilis* fructose-6-phosphate isomerase ManA is encoded by the open reading frame of the *B. subtilis manA* gene (Gen Bank accession number: NP_389084.1).

The *B. subtilis* fructose-6-phosphate isomerase ManA is encoded by the protein coding region of the *B. subtilis manA* gene (GenBank accession number:
(NP_389084.1). Thus, in an additional embodiment, the *Bacillus* cell has been genetically engineered to contain and express a nucleotide sequence which encodes the *B. subtilis* ManA or a functional variant thereof. Thus, in an additional and/or alternative embodiment, the *Bacillus* cell contains the *B. subtilis manA* gene or a functional variant thereof. In another additional and/or alternative embodiment, the *Bacillus* cell has been genetically engineered to contain and express a nucleotide sequence which encoded the *B. subtilis gmuF* gene or a functional variant thereof and/or a nucleotide sequence which encodes the *B. subtilis pmi* gene or a functional variant thereof.

In an additional and/or alternative embodiment, the expression of the native *manA* gene, the native *gmuF* gene and/or the native *pmi* gene can be increased. The expression of the native *manA* gene, the native *gmuF* gene and/or the native *pmi* gene can be increased in that the endogenous promoter of at least one of the native *manA* gene, the native *gmuF* gene and the native *pmi* gene is replaced by a stronger promoter, i.e. a promoter mediating an increased expression as compared to the native promoter of the *manA* gene, the *gmuF* gene and the *pmi* gene respectively. In another additional or alternative embodiment, the expression of the native *manA* gene, the native *gmuF* gene and/or the native *pmi* gene can be enhanced in that additional copies of the native *manA* gene, the native *gmuF* gene and/or the native *pmi* gene are propagated in the *Bacillus* cell.

In another additional or alternative embodiment, the expression of the native *manA* gene can be enhanced by deletion or functional inactivation of the *manP* gene encoding a mannose transporter leading to a constitutive expression of the native *manA* gene.

In another additional or alternative embodiment, the expression of the native *gmuF* gene can be enhanced by deletion or functional inactivation of the *gmuR* gene encoding the corresponding transcriptional repressor.

For expression of the fructose-6-phosphate isomerase, the *Bacillus* cell contains a recombinant fructose-6-phosphate isomerase gene, wherein the nucleotide sequence which encodes the fructose-6-phosphate isomerase is operably linked to expression control sequences.

The recombinant fructose-6-phosphate isomerase gene may be integrated into the *Bacillus* chromosome or present as an episomal version on a plasmid within the *Bacillus* cell.

In the second step of the *de novo* GDP-fucose biosynthesis, mannose-6-phosphate is converted to mannose-1-phosphate by the enzymatic activity of a phosphomannomutase. In an additional and/or alternative embodiment, the *Bacillus* cell has been genetically engineered to contain and express a nucleotide sequence which encodes a phosphomannomutase.

A suitable phosphomannomutase is the *E. coli* phosphomannomutase ManB. Thus, in an additional embodiment, the *Bacillus* cell has been genetically engineered to contain and express a nucleotide sequence which encodes *E. coli* ManB or a functional variant thereof.

The *E. coli* phosphomannomutase ManB is encoded by the nucleotide sequence of the protein coding region of the *E. coli manB* gene (Gen Bank accession number: NP_416552.1). Thus, in an additional and/or alternative embodiment, the *Bacillus* cell contains the *E. coli* manB gene or a functional variant thereof.

In an additional and/or alternative embodiment, the codon usage of the nucleotide sequence encoding the phosphomannomutase is adapted to the codon usage of *Bacillus.*

For expression of the phosphomannomutase, the *Bacillus* cell contains a recombinant phosphomannomutase gene, wherein the nucleotide sequence which encodes the phosphomannomutase is operably linked to expression control sequences.

The recombinant phosphomannomutase gene may be integrated into the *Bacillus* chromosome or present in the *Bacillus* cell as an episomal version on a plasmid. The next step in the *de novo* biosynthesis pathway of GDP-fucose is the formation of GDP-mannose by the following reaction:
α -D-mannose-1-phosphate + GTP + H⁺ => diphosphate + GDP-α-D-mannose

The conversion of mannose-1-phosphate to GDP-mannose is mediated by a GDP:mannose-1-phosphate guanylyltransferase. Thus, the Bacillus cell for producing a fucosylated oligosaccharide possesses a heterologous GDP:mannose-1-phosphate guanylyltransferase. A suitable GDP:mannose-1-phosphate guanylyltransferase is *E. coli* ManC or a functional variant thereof.

In an additional and/or alternative, the *Bacillus* cell has been genetically engineered to contain and express a nucleotide sequence which encodes a GDP:mannose-1-phosphate guanylyltransferase. An exemplary nucleotide sequence is the nucleotide sequence which encodes *E. coli* ManC. Thus, in an additional embodiment, the *Bacillus* cell has been genetically engineered to contain and express a nucleotide sequence which encodes the *E. coli* ManC or a functional variant thereof.

The *E. coli* GDP:mannose-1-phosphate guanylyltransferase ManC is encoded by the open reading frame of the *E. coli man*C gene (Gen Bank accession number: NP_416553.1). Thus, in an additional and/or alternative embodiment, the *Bacillus* cell contains the *E. coli man*C gene or a functional variant thereof.

In an additional and/or alternative embodiment, the codon usage of the nucleotide sequence encoding the GDP:mannose-1-phosphate guanylyltransferase is adapted to the codon usage of *Bacillus.*

For expression of the GDP:mannose-1-phosphate guanylyltransferase, the *Bacillus* cell contains a recombinant GDP:mannose-1-phosphate guanylyltransferase gene, wherein the nucleotide sequence which encodes the GDP:mannose-1-phosphate guanylyltransferase is operably linked to expression control sequences.

The recombinant GDP:mannose-1-phosphate guanylyltransferase gene may be integrated into the *Bacillus* chromosome or present as an episomal version on a plasmid.

In the subsequent step of the *de novo* GDP-fucose pathway, catalyzed by a GDP-mannose-4,6-dehydratase, GDP-D-mannose is converted to GDP-4-dehydro-6-deoxy-D-mannose.

Thus, the *Bacillus* cell for producing a fucosylated oligosaccharide possesses a GDP-mannose-4,6-dehydratase. A suitable GDP-mannose-4,6-dehydratase is *E. coli* Gmd.

In an additional and/or alternative, the *Bacillus* cell has been genetically engineered to contain and express a nucleotide sequence which encodes a GDP-mannose-4,6-dehydratase. An exemplary nucleotide sequence is the nucleotide sequence which encodes *E. coli* Gmd. Thus, in an additional embodiment, the *Bacillus* cell has been genetically engineered to contain and express a nucleotide sequence which encodes the *E. coli* Gmd or a functional variant thereof.

The *E. coli* GDP-mannose-4,6-dehydratase Gmd is encoded by the open reading frame of the *E. coli gmd* gene (Gen Bank accession number: NP_416557.1). Thus, in an additional and/or alternative embodiment, the *Bacillus* cell contains the *E. coli gmd* gene or a functional variant thereof.

In an additional and/or alternative embodiment, the codon usage of the nucleotide sequence encoding the GDP-mannose-4,6-dehydratase is adapted to the codon usage of *Bacillus.*

For expression of the GDP-mannose-4,6-dehydratase, the *Bacillus* cell contains a recombinant GDP-mannose-4,6-dehydratase gene, wherein the nucleotide sequence which encodes the GDP-mannose-4,6-dehydratase is operably linked to expression control sequences.

The recombinant GDP-mannose-4,6-dehydratase gene may be integrated into the *Bacillus* chromosome or present as an episomal version on a plasmid.

In the final reaction of the *de novo* GDP-fucose biosynthesis pathway, GDP-4-dehydro-6-deoxy-D-mannose is converted to GDP-fucose by a two-step NADPH-dependent reaction. This conversion is mediated by the GDP-fucose synthase and involves an epimerase reaction and a reductase reaction. Said epimerase reaction converts GDP-4-keto-6-deoxymannose to GDP-4-keto-6-deoxygalactose, which is then reduced to GDP-fucose.

Thus, the *Bacillus* cell for producing a fucosylated oligosaccharide possesses a GDP-fucose synthase. A suitable GDP-fucose synthase is E. coli WcaG or a functional variant thereof.

In an additional and/or alternative, the *Bacillus* cell has been genetically engineered to contain and express a nucleotide sequence which encodes a GDP-fucose synthase. An exemplary nucleotide sequence is the nucleotide sequence which encodes *E. coli* WcaG. Thus, in an additional embodiment, the *Bacillus* cell has been genetically engineered to contain and express a nucleotide sequence which encodes the *E. coli* WcaG or a functional variant thereof.

The *E. coli* GDP-fucose synthase WcaG is encoded by the open reading frame of the *E. coli wca*G gene (Gen Bank accession number: NP_416556.1). Thus, in an additional and/or alternative embodiment, the *Bacillus* cell contains the *E. coli wca*G gene or a functional variant thereof.

In an additional and/or alternative embodiment, the codon usage of the nucleotide sequence encoding the GDP-fucose synthase is adapted to the codon usage of *Bacillus.*

For expression of the GDP-fucose synthase, the *Bacillus* cell contains a recombinant GDP-fucose synthase gene, wherein the nucleotide sequence which encodes the GDP-fucose synthase is operably linked to expression control sequences. The recombinant GDP-fucose synthase gene may be integrated into the *Bacillus* chromosome or present as an episomal version on a plasmid.

In an additional and/or alternative embodiment, the *Bacillus* cell possesses a GDP-fucose salvage pathway for the intracellular biosynthesis of GDP-fucose. In the salvage pathway of GDP-L-fucose, free cytosolic fucose is phosphorylated by L-fucokinase to form L-fucose-L-phosphate, which is then further converted to GDP-L-fucose.

The salvage pathway for biosynthesis of GDP-fucose comprises the following enzymes:
I. a fucose kinase; and
II. a L-fucose-1-phosphate guanylyltransferase.

Hence, the *Bacillus* cell for producing a fucosylated oligosaccharide which possesses a GDP-fucose salvage pathway possesses a fucose kinase and a L-fucose-1-phosphate guanylyltransferase.

In an additional and/or alternative embodiment, the *Bacillus* cell has been genetically engineered to possess a fucose kinase and a L-fucose-1-phosphate guanylyltransferase.

The fucose kinase, also called fucokinase, ATP:6-deoxy-L-galactose 1-phosphotransferase, ATP:β-L-fucose 1-phosphotransferase or L-fucokinase activity, L-fucose kinase activity catalyzes the reaction L-fucose + ATP → β-L-fucose-1-phosphate + ADP + 2 H⁺.

The L-fucose-1-phosphate guanylyltransferase or GDP-L-fucose pyrophosphorylase then converts said β-L-fucose-1-phosphate to GDP-L- fucose.

In an additional and/or alternative embodiment, the *Bacillus* cell has been genetically engineered to contain and express a nucleotide sequence which encodes a fucose kinase or a functional variant thereof, and a L-fucose-1-phosphate guanylyltransferase or a functional variant thereof.

In an additional and/or alternative embodiment, the fucose kinase and the L-fucose-1-phosphate guanylyltransferase are combined in a single polypeptide. Suitable genes for transformation, coding for a fucose kinase, a fucose-1-phosphate guanylyltransferase and/or a bifunctional L-fucose-1-phosphate guanylyltransferase can be obtained from the genera *Bacteroides, Lentisphaera, Ruminococcus, Solibacter, Arabidopsis*, *Oryza, Physcomitrella, Vitis, Danio, Bos, Equus,* Macaca, *Pan, Homo, Rattus, Mus* and *Xenopus.* An example for a bifunctional fucose kinase/L-fucose-1-phosphate guanylyltransferase is found in *Bacterioides fragilis.*

In *B. fragilis,* the bifunctional fucose kinase/L-fucose-1-phosphate guanylyltransferase Fkp is encoded by the *B. fragilis* fkp gene (GenBank accession number AY849806).

In an additional and/or alternative embodiment, the *Bacillus* cell has been genetically engineered to contain and express a nucleotide sequence that encodes the *B. fragilis* Fkp or a functional variant thereof.

In an additional and/or alternative embodiment, the codon usage of the nucleotide sequences encoding the fucose kinase, fucose-1-phosphateguanylyltransferase and/or the bifunctional fucose kinase/L-fucose-1-phosphate guanylyltransferase is adapted to the codon usage of *Bacillus.*

For expression of the fucose kinase and the fucose-1-phosphateguanylyltransferase, the *Bacillus* cell contains at least one recombinant gene, wherein the protein-coding region encoding the fucose kinase and the L-fucose-1-phosphate guanylyltransferase and/or the bifunctional fucose kinase/L-fucose-1-phosphate guanylyltransferase fucose kinase is operably linked to expression control sequences.

The recombinant fucose kinase gene, fucose-1-phosphateguanylyltransferase gene and/or the bifunctional fucose kinase/L-fucose-1-phosphate guanylyltransferase gene may be integrated into the *Bacillus* chromosome or present as an episomal version on a plasmid.

For the intracellular formation of fucosyllactose, the *Bacillus* cell possesses a fucosyltransferase. The enzymatic activity of the fucosyltransferase transfers the fucose moiety from the donor substrate to the acceptor substrate. For the biosynthesis of a fucosyllactose, said acceptor substrate is lactose. Hence, the fucosyltransferase is a lactose-accepting fucosyltransferase.

The fucosyltransferase is selected from the group consisting of α-1,2-fucosyltransferases for the biosynthesis of 2'-fucosyllactose, and α-1,3-fucosyltransferases for the biosynthesis of 3-fucosyllactose.

In an additional and/or alternative, the *Bacillus* cell has been genetically engineered to contain and express at least one nucleotide sequence which encodes a fucosyltransferase.

For producing 2'-fucosyllactose (2'-FL), the α-1,2-fucosyltransferases WbgL from *E. coli* 0126 and FucT2 from *Helicobacter pylori* (EP 2 479 263 B1), the α-1,2-fucosyltransferases WblA from *Vibrio cholera* O22, FutD from *H. bilis* ATCC 437879, FutE from *H. cinaede* CCUG 18818, FutN from *Bacteroides vulgatus* ATCC 8482, FutO from *Bacteroides ovatus* ATCC 8483, WbgN from *E. coli* O55:H7, Bft1 and Bft3 from *Bacteroides fragilis* NCTC 9343 (WO 2014/018596 A2), and the α-1,2-fucosyltransferases FucT2 from *H. pylori* for the synthesis of Lewis Y and Lewis B saccharides (US 6,670,160 B2) were described and are suitable α-1,2-fucosyltransferases for 2'-FL biosynthesis in *Bacillus* cells.

For producing 3-fucosyllactose, the α-1,3-fucosyltransferase Amuc from *Akkermansia muciniphila,* and FucT6 and FucT7 from *Bacteroides fragilis* (EP 2 439 264 A1), the α-1,3-fucosyltransferase FutA from *H. pylori* (US 2014/ 0120611 A1) are described and are suitable α-1,3-fucosyltransferases for 3-FL biosynthesis in *Bacillus* cells. In addition, WO 2016/040531 A1 discloses the α-1,3-fucosyltransferase CafC from *B. nordii* CL02T12C05 for the synthesis of 3-fucosyllactose and lactodifucotetraose, and CafD from *H. hepaticus* ATCC51449 for the production of LN*n*FP-III.

Additional fucosyltransferases that can be expressed in *Bacillus* cells for the production of fucosylated saccharides are disclosed in WO 2019/0088133 A1, which is incorporated herein by reference.

In an additional and/or alternative embodiment, the codon usage of the nucleotide sequence encoding the fucosyltransferase is adapted to the codon usage of *Bacillus.*

For expression of the fucosyltransferase, the *Bacillus* cell contains at least one recombinant gene, wherein the protein-coding region encoding the fucosyltransferase is operably linked to expression control sequences.

The recombinant fucosyltransferase gene may be integrated into the *Bacillus* chromosome or present as an episomal version on a plasmid.

In an additional and/or alternative embodiment, the *Bacillus* cell for producing a fucosylated oligosaccharide is able to internalize L-fucose. The capability of internalizing L-fucose is of advantage for *Bacillus* cells which possess the GDP-fucose salvage pathway.

In an additional and/or alternative embodiment, the *Bacillus* cell has been genetically engineered to enable or improve internalization of L-fucose. Thus, the *Bacillus* cell possesses a heterologous L-fucose permease. A suitable L-fucose permease is *E. coli* FucP or a functional variant thereof.

In an additional and/or alternative embodiment, the *Bacillus* cell as been genetically engineered to contain and express a nucleotide sequence which encodes a L-fucose permease, preferably a nucleotide sequence that encodes the *E. coli* FucP or a functional variant thereof.

The E. coli L-fucose permease is encoded by the protein coding region of the *E. coli fuc*P gene (Gen Bank accession number: NP_417281.1). Hence, the *Bacillus* cell has been genetically engineered to contain and express a nucleotide sequence that encodes the *E. coli* FucP or a functional variant thereof.

In an additional and/or alternative embodiment, the codon usage of the nucleotide sequence encoding the L-fucose permease is adapted to the codon usage of *Bacillus.*

For expression of the L-fucose permease, the *Bacillus* cell contains a recombinant L-fucose permease gene, wherein the nucleotide sequence which encodes the L-fucose permease is operably linked to expression control sequences.

The recombinant L-fucose permease gene may be integrated into the *Bacillus* chromosome or present as an episomal version on a plasmid.

In an additional and/or alternative embodiment, the *Bacillus* cell for producing a fucosylated oligosaccharide lacks any β-galactosidase activity or possesses reduced β-galactosidase activity as compared to a wild type *Bacillus* cell of the same species.

The intracellular biosynthesis of fucosylated oligosaccharides requires import of lactose as acceptor substrate for the lactose-accepting fucosyltransferase. Any intracellular enzyme activity that hydrolyzes internalized lactose would affect the efficacy of fucosyllactose formation as the pool of intracellular lactose would be diminished. Therefore, it would be advantageous if the *Bacillus* cell for producing a fucosylated oligosaccharide would lack or at least possess - as compared to the wild-type *Bacillus* cell - reduced beta-galactosidase activity.

In an additional and/or alternative embodiment, the *Bacillus* cell has been genetically engineered to abolish or at least reduce the cell's β-galactosidase activity.

In an additional and/or alternative embodiment, the *Bacillus* cell has been genetically engineered by deletion or functional inactivation of the *gan*A gene. In another embodiment, the *Bacillus* cell has been genetically engineered to reduce the expression level of the *gan*A gene - as compared to the wild-type *Bacillus* cell.

The *Bacillus gan*A gene is also called *yvf*N or *lac*A. It is a gene of the GanR regulon which contains genes encoding enzymes involved in the utilization of galactan. The *gan*A gene encodes a beta-galactosidase that is involved in the galactan utilization of *Bacillus.*

Deletion or functional inactivation of the *gan*A gene abolishes the GanA-mediated β-galactosidase activity in the *Bacillus* cell, whereas reduction of the *ganA* expression lowers the amount of GanA in the *Bacillus* cell and hence the β-galactosidase activity which could interfere with the biosynthesis of a fucosylated oligosaccharide.

In an additional and/or alternative embodiment, the *Bacillus* cell has been genetically engineered by deletion or functional inactivation of the *yesZ* gene. The *Bacillus yes*Z gene encodes the beta-galactosidase YesZ which plays a role in the degradation of rhamnogalacturonan being derived from plant cell walls. The expression of *Bacillus yes*Z gene is induced by rhamnogalacturonan I. In another embodiment, the *Bacillus* cell has been genetically engineered to reduce the expression level of the *yesZ* gene - as compared to the wild-type *Bacillus* cell.

Deletion or functional inactivation of the *yesZ* gene abolishes the YesZ-mediated β-galactosidase activity in the *Bacillus* cell, whereas reduction of the *yesZ* expression lowers the amount of YesZ in the *Bacillus* cell and hence the β-galactosidase activity which could interfere with the biosynthesis of a fucosylated oligosaccharide. When *B. subtilis* enters the post-exponential growth phase, they (start to) produce large quantities of extracellular proteases. Foreign proteins are often protease sensitive. Therefore, an exoprotease-free strain is desirable to increase the stability of heterologous proteins and to allow accumulation of high levels of foreign proteins. The genome of *Bacillus* encodes for at least eight extracellular proteases, namely *nprE, aprE, epr, bpr, mpr, nprB, vpr* and *wprA.* Thus, in an additional and/or alternative embodiment, the *Bacillus* cell has been genetically engineered in that at least one gene encoding for an extracellular protease has been deleted or functionally inactivated, preferably at least one of the genes selected from the group consisting of *nprE, aprE, epr, bpr, mpr, nprB, vpr* and *wprA.* Preferably, two, three, four, five six, seven or eight of the genes selected from the group consisting of *nprE, aprE, epr, bpr, mpr, nprB, vpr* and *wprA* have been deleted or functionally inactivated.

*B. subtilis* synthesizes pulcherriminic acid when growing in media containing a carbohydrate such as glucose or lactose. Excreted pulcherriminic acid forms the red pigment pulcherrimin, the salt of pulcherriminic acid (a ferric chelate), when iron is present in the growth medium. The formation of this undesired by-product during fermentation processes can be avoided/abolished by deletion or disruption of the genes *yvmC* and/or *cypX.* The gene *yvmC* encodes a cyclodipeptide synthase and the gene *cypX* encodes a cytochrome P450 cyclo-l-leucyl-l-leucyl dipeptide oxidase.

Thus, in an additional and/or alternative embodiment, the *Bacillus* cell has been genetically engineered in that at least one of the genes *yvmC* and *cypX* has been deleted or functionally inactivated.

The rhizobacterium *B. subtilis* possesses genes for the synthesis of more than 20 antibiotics. Among them are peptide antibiotics like *Bacillus subtilis* lantibiotics and lantibiotic-like peptides (subtilin, ericin S, mersacidin, sublancin 168, subtilosin A) and non-ribosomally synthesized (peptide) antibiotics (surfactin, iturin, bacillomycin, mycosubtilin, corynebactin/ bacillibactin, fengycin plipastatin, mycobacillin, TL-119, bacilysin, bacilysocin, amicoumacin, 3,3'-neotrehalosadiamine, difficidin, rhizocticin).

For producing a fucosylated oligosaccharide, it is preferred to use a *Bacillus* cell that does not produce an antibiotic. Thus, in an additional and/or alternative embodiment, the *Bacillus* cell does not synthesize one or more of the antibiotics selected from the group consisting of lantibiotics and lantibiotic-like peptides such as subtilin, ericin S, mersacidin, sublancin 168, subtilosin A; non-ribosomally synthesized (peptide) antibiotics such as surfactin, iturin, bacillomycin, mycosubtilin, corynebactin/ bacillibactin, fengycin plipastatin, mycobacillin, TL-119, bacilysin, bacilysocin, amicoumacin, 3,3'-neotrehalosadiamine, difficidin and rhizocticin. The *Bacillus* cell may have been genetically engineered to obtain a *Bacillus* cell that does not synthesize one or more of said antibiotics.

Wild-type *Bacillus* cells are capable of forming spores. Sporulation, i.e. the process of forming spores, in bacteria is considered a reaction of the bacterial cell which initiates a developmental program leading to the formation of daughter cells of distinct morphology and fate. Sporulation of *Bacillus* was studied as a basic model for cell differentiation. During sporulation, a rod-shaped *Bacillus* cell divides asymmetrically, thereby resulting in two genetically identical cells with different morphology and fates.

However, in industrial production it is not desired if the bacterial production strain sporulates during fermentation. Therefore, it is preferred to use *Bacillus* cells for the production of fucosylated oligosaccharides that are not capable of forming spores. Such *Bacillus* cells are called "non-sporulating".

Preferably, the non-sporulating *Bacillus* cell capable to produce a fucosylated oligosaccharide originated from one of the B. *subtilis* strains listed in Table 1.

**Table 1: Non-limiting list of Bacillus subtilis strains.**

| **Strain** | **Manufacturer/Reference** | **Product** | **Application** |
|---|---|---|---|
| *Bacillus subtilis* 168 | BGSCID 1A1 | - | reference strain |
| *Bacillus subtilis* 168 *ΔspollGA::erm* | BGSCID BKE15310 | - | reference strain |
| *Bacillus subtilis* 168 *ΔsigE::erm* | BGSCID BKE15320 | - | reference strain |
| *Bacillus subtilis* 168 *ΔsigG::erm* | BGSCID BKE15330 | - | reference strain |
| *Bacillus subtilis* 168 Δ*sigF::erm* | BGSCID BKE23450 | - | reference strain |
| *Bacillus subtilis* 3NA | BGSCID 1S1 | - | reference strain |
| *Bacillus subtilis* W23 | BGSCID 2A9 | - | reference strain |
| *Bacillus subtilis* PY79 | BGSCID 1A747 | - | reference strain |
| *Bacillus subtilis* | Garden of Life® | Primal Defense® | Human use |
| *Bacillus subtilis* | Roux-Ocefa Laboratorios | Totalflora | Human use |
| *Bacillus subtilis* | Ist. Bioch. Italiano | Lactipan Plus | Human use |
| *Bacillus subtilis* 2335 | Biopharma | Biosporin® | Human use |
| LifeinU™ *Bacillus subtilis* CU1 | Lesaffre Human Care | Super Smart® | Human use |
| *Bacillus subtilis* PXN/21 | Probiotics International Ltd | Bio-Kult® | Human use |
| *Bacillus subtilis* HU58 | Microbiome LABS | HU58™ | Human use |
| *Bacillus subtilis* R0179 | Hanmi Pharmaceutical Co. Ltd. | Medilac® | Human use |
| *Bacillus subtilis* DE111 | Deerland Enzymes & Probiotics | NutriCommit | Human use |
| *Bacillus subtilis* DSM21097 | MorinagaGarden B.V. | Morinaga Plus⁺ | Human use |
| *Bacillus subtilis* IDCC1101 | Ildong Pharmaceutical | Biobaby® | Human use |
| *Bacillus subtilis* KATMIRA1933 | JSL Foods | YoguFarm | Human use |
| *Bacillus subtilis* spp. Natto | *various* | Natto | Human use |
| *Bacillus subtilis* SC-8 | *various* | Cheonggukjang | Human use |
| *Bacillus subtilis* CH201/DSM5750 | Christian Hansen | BioPlus2B® | Veterinary Use |
| *Bacillus subtilis* PB6 | Kenim® | CLOSTAT® | Veterinary Use |

In an additional and/or alternative embodiment, the *Bacillus* cell has been genetically engineered by deletion or functional inactivation of SpoOA. Suitable functional inactivation of Spo0A includes deletion of the phosphorylation site, where SpoOF and Spo0B phosphotransferases phosphorylate SpoOA.

In an additional and/or alternative embodiment, the *Bacillus* cell has been genetically engineered by deletion or functional inactivation of the genes encoding sigma factor SigE (*sigE*) and/or sigma factor SigF (*sigF*).

The *Bacillus* cell of the invention is able to produce a fucosylated oligosaccharide when cultivated in the presence of lactose in a medium and under conditions that are permissive for the *Bacillus* cell to produce the fucosylated oligosaccharide.

According to the second aspect, provided is the use of a *Bacillus* cell as described herein before for the production of a fucosylated oligosaccharide, preferably a fucosylated human milk oligosaccharide such as 2'-FL, 3-FL or 2',3-DiFL.

Since *Bacillus* is generally recognized as safe, production of fucosylated oligosaccharides for human consumption utilizing a production organism that is considered as generally safe should be considered safe too, provided that the amendments to the *Bacillus* cells for producing the fucosylated oligosaccharide does not affect the safety of the production strain with respect to human health and the environment. Hence, it is expected that there will be less concerns affecting regulatory approval of a fucosylated oligosaccharide for human consumption, in particular of fucosylated human milk oligosaccharides for human consumption, more specifically for infant formula, which consists of or contains a fucosylated oligosaccharide that has been produced by using a *Bacillus* cell as described herein. Thus, acceptance of microbially produced fucosylated oligosaccharides in infant formula and nutritional compositions at regulatory authorities as well as among consumers should be better.

According to the third aspect, provided is a method for the production of a fucosylated oligosaccharide the method comprises:
- providing a non-sporulating *Bacillus* cell that had been genetically engineered to possess a lactose permease, a GDP-fucose biosynthesis pathway, and a fucosyltransferase;
- cultivating the *Bacillus* cell in a culture medium containing lactose and under conditions that are permissive for the production of the fucosylated oligosaccharide, and
- optionally retrieving the fucosylated oligosaccharide form the culture medium and/or from the *Bacillus* cell.

The non-sporulating *Bacillus* cell that is provided is a *Bacillus* cell as described herein.

In an additional and/or alternative embodiment, the fucosylated oligosaccharide is selected from the group consisting of 2'-fucosyllactose (2'-FL), 3-fucosyllactose (3-FL), 2',3-difucosyllactose (DiFL), lacto-*N*-fucopentaose I (LNFP I), lacto-*N*-*neo*fucopentaose I (LNnFP I), lacto-*N*-fucopentaose II (LNFP II), lacto-*N*-fucopentaose III (LNFP III), lacto-*N*-fucopentaose V (LNFP V), lacto-*N*-*neo*fucopentaose V (LNnFP V), lacto-*N*-difucohexaose I (LNDH I) and lacto-*N*-difucohexaose II (LND).

In an additional and/or alternative embodiment, the culture medium contains L-fucose, in particular for cultivating a *Bacillus* cell for the production of a fucosylated oligosaccharide which possesses the GDP-fucose salvage pathway for providing GDP-fucose as donor substrate for the fucosyltransferase.

The invention also extends to fucosylated oligosaccharides that were produced by a *Bacillus* cell and/or a method as described herein, to the use of said fucosylated oligosaccharides for the manufacturing of a nutritional composition, preferably an infant formula, a dietary supplement or a medicinal food. Moreover, the present invention also extends to nutritional compositions containing a fucosylated oligosaccharide that was produced by a *Bacillus* cell and/or a method as described herein.

The present invention will be described with respect to particular embodiments and with reference to drawings, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding. The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

### Examples

### Example 1: Transformation of Bacillus subtilis

*Bacillus subtilis* can be genetically manipulated by various techniques. For transformation of *B. subtilis,* competent cells were prepared by a modified protocol of the two-step method (Anagnostopoulos, C. and Spizizen, J. (1961) J Bacteriol 81 (5): 741-746). An overnight culture was inoculated in MG1 medium and shaken at 37 °C. MG1 medium is Spizizen's minimal medium, which is supplemented with 0.5 % glucose, 5 mM MgSO₄ and 0.02 % casamino acids (optionally additionally supplemented with biotin and/or L-tryptophan). At the next morning, this culture was diluted 1:20 in fresh MG1 medium and incubated at 37 °C for approximately 6 h. 1 ml of the culture was diluted in 8 ml MG2 medium which differs from MG1 medium in the concentration of casamino acids (0.01 % instead of 0.02 %). In a shortened protocol the overnight culture is directly diluted in MG2 medium. After incubation for another 90 min, a 1-ml portion of the culture was mixed with 1-3 µg multimeric plasmid DNA or linear DNA and incubated at 37 °C for 30-60 min with shaking. Multimeric plasmid DNA was obtained either by using *E. coli* strain NM538 for propagation of plasmid DNA or by linearization of the plasmid by digestion with a single-cutter restriction enzyme, which cleaves within the backbone, followed by re-ligation with T4 DNA ligase.

Afterwards, cells were spread on 2x YT agar plates containing the appropriate antibiotic. Antibiotics were added in the following concentrations: 5 µg·mL⁻¹ erythromycin, 5 µg·mL⁻¹ chloramphenicol, 10 µg·mL⁻¹ kanamycin,100 µg·mL⁻¹ spectinomycin.

Alternatively, for protoplast transformation (Romero, D. et al. (2006) Journal of Microbiological Methods 66:556-559), cells were grown in 20 ml of Penassay broth (PAB) at 37 °C until the onset of the stationary phase of growth (OD₆₀₀=1.7-2). Cells were then pelleted and resuspended in 10 ml of SMPP medium (0.3% bovine serum albumin, 5% 2 M sucrose, 25% 4x PAB, 50% 2x SMM), composition of 2x SMM being 1 M sucrose, 0.04 M maleic acid disodium salt hydrate and 0.04 M MgCl₂ (pH 6.5). After the addition of lysozyme (10 mg ml⁻¹) and mutanolysin (75 U ml⁻¹) the mixture was incubated at 37 °C with shaking to generate protoplasts. Protoplast formation was checked microscopically. Protoplasts were then carefully harvested by centrifugation at 5200 xg and 4 °C for 5 min, washed twice with ice cold washing-electrotransformation buffer (1× SMM) and finally suspended in this solution. Plasmid DNA (1-3 µg) was added to 120 µl of protoplast suspension and the mixture was kept on ice for at least 5 min. The transformation mixture was transferred to a 0.2-cm cuvette and a single electroporation pulse was applied at 25 µF, 400 Ω and 0.7 kV. Immediately after the electroporation shock 1 ml of recovering medium (equal volumes of 4x PAB and 2x SMM, prepared freshly before use) was added to the cuvette. The transformation reaction was then transferred to a 2 ml tube and incubated at 37 °C with shaking for 12 h. For regeneration, the cell suspension was spread on DM3 agar plates (Chang, S. and Cohen, S. (1979) MGG 168(1):111-115) and incubated at 37 °C for 48 h. DM3 regeneration medium contained the following sterile solutions per liter: 200 ml 4% agar, 100 ml 5% casamino acids, 50 ml 10% yeast extract, 100 ml 3.5% K₂HPO₄ and 1.5% KH₂PO₄, 25 ml 20% glucose, 20 ml 1 M MgCl₂, 500 ml 0.5 M sorbitol and 5 ml filter sterilized 2% bovine serum albumin (added to the mixture when the temperature is below 55 °C) and was supplemented with the appropriate antibiotic.

Electroporation of *B. subtilis* was done according to a modified protocol from Zhang et al. (2011) provided by MoBiTec GmbH (Zhang,G., Bao,P., Zhang,Y., Deng,A., Chen,N. and Wen,T. (2011) Anal. Biochem., 409:130-137). A 2x YT overnight culture was diluted 100-fold with fresh 2x YT medium and the culture was grown to an OD₆₀₀ of 0.2 at 37 °C on a rotary shaker. Then the culture was supplemented with 1% DL-threonine, 2% glycine, 0.1% tryptophan and 0.03% Tween 80. After cultivation for another 60 min, the cell suspension was cooled on ice for 20 min, centrifuged at 5000 x g for 10 min at 4 °C and washed twice with electroporation buffer (0.5 M trehalose, 0.5 M sorbitol, 0.5 M mannitol, 0.5 mM MgCl₂, 0.5 mM K₂HPO₄, 0.5 mM KH₂PO₄, pH 7.4, filter-sterilized and stored frozen). Finally, cells were resuspended in electroporation buffer at 1/100 of the original culture volume and 100 µl of cell suspension was mixed with DNA. The transformation mixture was transferred to a 0.1-cm cuvette and electroporation was performed at 1.8 kV with a single pulse delivered with a MicroPulser™ device (Bio-Rad). Immediately after pulse delivery, 1 ml 2x YT broth containing 0.5 M sorbitol and 0.38 M mannitol was added to the cuvette. The transformation suspension was transferred to a 2 ml tube and incubated at 37 °C for 3 h on a rotary shaker. The cells were spread on a selective 2x YT agar plate and incubated at 37 °C overnight.

Using an alternative electroporation protocol (Xue, G. P., J. S. Johnson, and B. P. Dalrymple: 1999; Journal of Microbiological Methods 34:183-191), 5 ml of LB containing 0.5 M glucitol was inoculated with *B. subtilis* and incubated overnight at 37 °C. Subsequently, the overnight culture was diluted (1:16) by 75 ml of LB containing 0.5 M glucitol and incubated until an OD₆₀₀ of 0.85 - 0.95 was obtained. The cells were then pelleted by centrifugation for 10 min at 4 °C at 5.000xg and washed four times by ice-cooled electroporation buffer (10 % glycerol, 0.5 M glucitol, 0.5 M mannitol). Finally, the cells were resuspended in 1-2 ml electroporation buffer. Electroporation was performed using 60 µl of competent cells with DNA in a cooled electroporation cuvette (1-mm electrode gap). The cell-DNA mixture was subjected to a single electrical pulse at 25 µF, 200 Ω and 21 kV/cm. Finally, 1 ml recovery broth (LB containing 0.5 M glucitol and 0.38 mannitol) was added to the electropermeabilized cells and the bacterial culture was incubated for 3 h at 37 °C followed by plating on the LB agar supplemented by an antibiotic.

Two different rich media were used, namely Luria Broth (LB) and 2x YT. Luria Broth (LB) medium consisted of 1% tryptone, 0.5% yeast extract and 0.5% NaCl (pH 7.2).

2x YT medium consisted of 1.6% tryptone, 1% yeast extract and 0.5% NaCl (pH 7.5).

To prepare rich-medium agar plates 15 g L⁻¹ agar were added.

For shaking flask experiments, Spizizen's minimal medium (Spizizen, J. 1958 Proc. Natl. Acad. Sci. U. S. A. 44(10):1072-1078) was used.

Spizizen's minimal medium contains the following salts: 2 g/L (NH₄)₂SO₄, 14 g/L K₂HPO₄, 6 g/L KH₂PO₄, 1 g/L Na₃ citrate × 2·H₂O and 0.2 g/L MgSO₄ × 7·H₂O.

The preculture medium consisted of Spizizen's minimal salts supplemented with 2 % D-glucose, 0.05 % casamino acids and MgSO₄ to a final concentration of 2 mM (optionally additionally supplemented with biotin and/or L-tryptophan).

The main culture medium consisted of Spizizen's minimal salts supplemented with 2 % D-glucose, 0.05 % casamino acids, MgSO₄ to a final concentration of 2 mM and 0.5 mL·L⁻¹ 1000x trace element solution (optionally additionally supplemented with biotin and/or L-tryptophan).

Trace element solution (1000x) consisted of 100.6 g L⁻¹ C₆H₉NO₆, 56.4 g·L⁻¹ ammonium ferric citrate, 9.8 g·L⁻¹ MnCl₂ × 4·H₂O, 1.6 g·L⁻¹ CoCl₂ × 6·H₂O, 1 g·L⁻¹ CuCl₂ × 2·H₂O, 1.9 g·L⁻¹ H₃BO₃, 9 g·L⁻¹ ZnSO4 × 7·H₂O, 1.1 g·L⁻¹ Na₂MoO₄ × 2·H₂O, 1.5 g·L⁻¹ Na₂SeO₃, 1.5 g·L⁻¹ NiSO₄ × 6·H₂O.

If necessary, the appropriate antibiotic(s) were added to the medium to make it selective.

*B. subtilis* strains were initially grown on rich-media agar plates to obtain single colonies. These plates were grown for 1 day at 30-37°C. For shaking flask experiments, a 20-ml preculture was inoculated with a single colony and grown over night at 30-37 °C on a rotary shaker. Subsequent 20-ml main cultures were inoculated with this preculture to a starting OD₆₀₀ of about 0.1 and incubated at 30-37 °C on a rotary shaker. If induction was required, a 40-60 ml main culture was splitted into 20-ml proportions at the timepoint of induction. The culture volume did not exceed 20 % of the shaking flask capacity.

### Example 2: Construction of a Bacillus subtilis production strain for 2'-fucosyllactose

Metabolic engineering of a sporulation-deficient *Bacillus subtilis* strain (table 1) was achieved by the integration of the heterologous genes *E. coli manC, E. coli manB* and E. *coli manA* and simultaneous deletion of the endogenous gene *lacA* by homologous recombination. The B. *subtilis* gene *ganA* (*yvfN, lacA),* which is positioned within the galactan operon, encodes a beta-galactosidase.

For synthesis of GDP-mannose the open reading frames of *manC, manB* and *manA* were operably linked to the *B. subtilis* constitutive promoter P43 (iGem part repository: sequence ID: BBa_K143013) as an operon. The gene *manC* (Gen Bank accession number: NP_416553.1) encodes GDP:mannose-1-phosphate guanylyltransferase from *E. coli.* The gene *manB* (Gen Bank accession number: NP_416552.1) encodes *E. coli* phosphomannomutase and the gene *manA* (Gen Bank accession number: NP_389084.1) encodes *B. subtilis* fructose-6-phosphate isomerase. Each of the genes mentioned above was fused *in silico* to a *B. subtilis* RBS sequence. The herein described expression cassette *<P43-manCBA>* was codon-optimized for expression in *B. subtilis* and prepared synthetically by GenScript Corp. Subsequently, the complete integration cassette was assembled and cloned into pBR322 (New England Biolabs GmbH, Frankfurt, Germany) to create the suicide plasmid <pBR322 flank *ganA* up*-lox71-erm-lox66-*P43*-manCBA-*flank *ganA* down> (SEQ ID NO: 1). Afterwards, *B. subtilis* was transformed with this plasmid by its natural competence. Cells were spread on 2x YT agar plates containing the appropriate antibiotic (5 µg mL⁻¹ erythromycin). Integration of the expression cassette *<P43-manCBA>* into the *ganA* locus of the *B. subtilis* genome, yielding strain A, was verified by colony PCR. Gene expression was confirmed by targeted proteomics and/or by real-time PCR.

For the production of 2'-fucosyllactose from lactose and GDP-mannose in the resulting *<P43-manCBA>* integration strain, an expression plasmid (SEQ ID NO: 2) was constructed that comprises all necessary genes under the control of the inducible promoter P*_{grac100}*. Therefore, the *B. subtilis* expression vector pHT253 (MoBiTec GmbH, Göttingen, Germany) was used as backbone. The open reading frame of the *E. coli lacY* gene (Gen Bank accession number: NP_414877.1) was amplified by PCR from chromosomal DNA. The open reading frame of the gene *wbgL,* encoding for a *E. coli* O126 α-1,2-fucosyltransferases, was codon-optimized for expression in *B. subtilis* and prepared synthetically by GenScript Corp. Also, the open reading frames of the *E. coli* genes *gmd* (Gen Bank accession number: NP_416557.1; encoding for a GDP-mannose-4,6-dehydratase) and *wcaG* (Gen Bank accession number: NP_416556.1; encoding for a GDP-fucose synthase) were codon-optimized for expression in *B. subtilis* and prepared synthetically by GenScript Corp. Each gene enclosed in the inducible expression cassette was linked to a *B. subtilis* RBS sequence. Additionally, a suitable *B. subtilis* terminator sequence from the iGem part repository (sequence ID: BBa_B0015) was introduced in the final expression plasmid <pHT253 P*_{grac100}*-*wbgL-gmd- wcaG-lacY-terminator>* (SEQ ID NO: 2). The *<P43-manCBA>* integration strain was transformed with this expression plasmid (SEQ ID NO: 2) by its natural competence. Gene expression was confirmed by targeted proteomics and/or by real-time PCR. Transformants were cultivated under conditions that are permissive for the *B. subtilis* to produce 2'-fucosyllactose in the presence of exogenous lactose.

### Example 3: Construction of a Bacillus subtilis production strain for 2'-fucosyllactose

Strain A, which is described in example 2, was used as parent strain. For the production of 2'-fucosyllactose from lactose and GDP-mannose, additionally, the *E. coli* genes *lacY, gmd, wcaG* and *wbgL* were integrated in the endogenous *amyE* (*amyA*) locus of strain A (encoding for an alpha-amylase).

The open reading frame of the gene *wbgL* (encoding for a α-1,2-fucosyltransferase) was codon-optimized for expression in *B. subtilis* and prepared synthetically by GenScript Corp. Also, the open reading frames of the genes *gmd* (Gen Bank accession number: NP_416557.1; encoding for a GDP-mannose-4,6-dehydratase) and *wcaG* (Gen Bank accession number: NP_416556.1; encoding for a GDP-fucose synthase) were codon-optimized for expression in *B. subtilis* (carried out by GenScript Corp), placed under the control of the strong constitutive *B. subtilis* promoter P*_{lepA}* as a two-gene operon and prepared synthetically by GenScript Corp. The open reading frame of the *E. coli lacY* gene (Gen Bank accession number: NP_414877.1), encoding a lactose permease, was amplified by PCR from chromosomal DNA.

All necessary genes for the production of 2'-fucosyllactose from lactose and GDP-mannose were assembled to the constitutive expression cassette <P43*-wbgL-lacY-P_{lepA}-gmd-wcaG>* in which each gene was linked to a *B. subtilis* RBS sequence. Additionally, a suitable *B. subtilis* terminator sequence from the iGem part repository (sequence ID: BBa_B0015) was placed downstream of this expression cassette. The above-described construct was cloned into pBR322 (New England Biolabs GmbH, Frankfurt, Germany) to generate <pBR322 flank *amyE* up*-lox71-aad9-lox66-*P43*-wbgL-lacY-*P*_{lepA}-gmd wcaG-terminator-flank amyE* down> (SEQ ID NO: 3).

The resulting *amyE* integration vector (SEQ ID NO: 3) was used to transform *B. subtilis* by its natural competence. Transformants were selected by spectinomycin (100 µg ml⁻¹). Integration of the expression cassette <P43-*wbgL-lacY-P_{lepA}-gmd wcaG-terminator>* into the *amyE* locus of the *B. subtilis* genome was verified by colony PCR. Gene expression was confirmed by targeted proteomics and/or by real-time PCR.

### Example 4: Construction of a Bacillus subtilis production strain for 2'-fucosyllactose

For the production of 2'-fucosyllactose from GDP-fucose and exogenous lactose, initially, the *E. coli* gene *lac*Y was integrated into the endogenous *amyE* (*amyA*) locus (encoding for an alpha-amylase). The open reading frame of the *E. coli lacY* gene (Gen Bank accession number: NP_414877.1), encoding a lactose permease, was amplified by PCR from chromosomal DNA. The integration cassette <flank *amyE* up-*lox71-aad9-lox66-*P43*-lacY*-flank *amyE* down > (SEQ ID NO: 4) was constructed and cloned into pBR322 (New England Biolabs GmbH, Frankfurt, Germany). *B. subtilis* was transformed with the resulting plasmid by its natural competence. Integration of the expression cassette <P43-*lacY*> into the *amyE* locus of the *B. subtilis* genome, yielding strain B, was verified by colony PCR. Gene expression was confirmed by targeted proteomics and/or by real-time PCR.

For synthesis of GDP-fucose from exogenous L-fucose a *B. subtilis* expression plasmid was constructed, comprising all necessary genes. Plasmid pDW1 was used as backbone. This plasmid was prepared synthetically by GenScript Corp and contains the genes *bla* and *erm* for selection in *E. coli* and *B. subtilis,* respectively. Additionally, it contains the replicon of pMB1 for propagation in *E. coli* and the pUB110 replicon for propagation in *B. subtilis.*

The *B. fragilis* gene *fkp* (GenBank accession number AY849806) encodes for a bifunctional fucose kinase/L-fucose-1-phosphate guanylyltransferase, the *E. coli* gene *wbgL* encodes for a α-1,2-fucosyltransferases and the *E. coli* gene *fucP* (Gen Bank accession number: NP_417281.1) encodes for a L-fucose permease. The open reading frames of these genes were each codon-optimized for expression in *B. subtilis* and prepared synthetically by GenScript Corp. An expression cassette was constructed that comprises the genes *fkp, wbgL* and *fucP* under the control of the strong constitutive *B. subtilis* promoter P43.

Each gene enclosed in the expression cassette was linked to a *B. subtilis* RBS sequence. Additionally, a suitable *B. subtilis* terminator sequence from the iGem part repository (sequence ID: BBa_B0015) was introduced in the final expression plasmid <pDW1-P43*-fkp-wbgL-fucP-terminator*> (SEQ ID NO: 5). Strain B was transformed with this expression plasmid (SEQ ID NO: 5) by its natural competence. Gene expression was confirmed by targeted proteomics and/or by real-time PCR. Transformants were cultivated under conditions that are permissive for *B. subtilis* to produce 2'-fucosyllactose in the presence of exogenous lactose and L-fucose.

### Example 5: Production of 2'-fucosyllactose using metabolically engineered Bacillus subtilis strains

A preculture was inoculated with a metabolically engineered *Bacillus subtilis* strain suitable for the biosynthesis of 2'-fucosyllactose (as described in example 2, 3 and 4, respectively).

The preculture was incubated at 30-37 °C over night and then diluted to a starting OD₆₀₀ of about 0.1 in fresh main culture medium. When the main culture reached an OD₆₀₀ of approximately 0.5, 2 mM lactose was added to the growth medium. When the inducible promoter P*_{grac100}* was used for gene expression, induction was carried out with lactose (2 mM) or with both lactose (2 mM) and IPTG (1 mM). For the salvage biosynthesis of GDP-fucose, additionally, 2 mM L-fucose were added. Cultivation was discontinued after 24 h / 48 h and intracellular and extracellular 2'-fucosyllactose was analyzed by thin layer chromatography and/or HPLC and/or mass spectrometry (as described in WO 2017/042382 A or WO 2019/008133 A). Biosynthesis of substantial amounts of 2'-fucosyllactose was detected.

## Claims

1. A non-sporulating *Bacillus* cell for the production of a fucosylated oligosaccharide, wherein said cell has been genetically engineered to possess a lactose permease, a GDP-fucose biosynthesis pathway and a fucosyltransferase.

2. The *Bacillus* cell according to claim 1, wherein the sporulation has been impaired by deletion or functional inactivation of one or more of the genes encoding Spo0A, sigma E and sigma F.

3. The *Bacillus* cell according to claim 1 or 2, wherein the lactose permease is *E. coli* LacY or a functional variant thereof.

4. The *Bacillus* cell according to any one of claims 1 to 3, wherein the GDP-fucose biosynthesis pathway is a *de novo* GDP-fucose biosynthesis pathway and/or a salvage pathway.

5. The *Bacillus* cell according to any one of claims 1 to 4, wherein the fucosyltransferase is a lactose-accepting fucosyltransferase, preferably a fucosyltransferase selected from the group of α-1,2-fucosyltransferases and α-1,3-fucosoyltransferases.

6. The *Bacillus* cell according to any one of claims 1 to 5, wherein said *Bacillus* cell lacks any β-galactosidase activity or possesses reduced β-galactosidase activity as compared to a wild-type progenitor *Bacillus* cell of the same species.

7. The *Bacillus* cell according to claim 6, wherein the *Bacillus* cell has been genetically engineered by deletion or functional inactivation of at least one of the genes selected from the group consisting of *yesZ* and *ganA.*

8. The Bacillus cell according to any one of claims 1 to 7, wherein said *Bacillus* cell is a *Bacillus subtilis* cell.

9. Use of a *Bacillus* cell according to any one of claims 1 to 8 for the production of a fucosylated oligosaccharide.

10. A method for the production of a fucosylated oligosaccharide,
- providing a non-sporulating *Bacillus* cell as defined in any one of claims 1 to 8;
- cultivating said *Bacillus* cell in a medium and under conditions that are permissive for the production of a fucosylated oligosaccharide; and optionally
- retrieving the fucosylated oligosaccharide from the medium and/or the *Bacillus* cell.

11. The method according to claim 10, wherein the medium contains lactose.

12. The method according to claim 10 or 11, wherein the medium contains L-fucose.

13. A fucosylated oligosaccharide produced by a method according to any one of claims 10 to 12.

14. Use of a fucosylated oligosaccharide according to claim 13 for the manufacture of a nutritional composition, preferably of an infant formula.

15. A nutritional composition containing a fucosylated oligosaccharide according to claim 13.
